# EUROPEAN PATENT APPLICATION

(11) **EP 4 335 917 A1**
(43) Date of publication of application: **13.03.2024**
(21) Application number: 22799041.3
(22) Date of filing: 26.04.2022
(51) Int. Cl.: C12N 5/0775, A61K 35/28, A61P 19/00, A23L 33/10

(54) **MESENCHYMAL STEM CELLS HAVING ENHANCED OSTEOGENIC DIFFERENTIATION CAPACITY, AND USE THEREOF**

(30) Priority: 07.05.2021 KR 20210059516; 25.04.2022 KR 20220051044
(71) Applicant: Sungkwang Medical Foundation, Seoul 06135 (KR)
(72) Inventor: RIM, Jong Seop, Seongnam-si, Gyeonggi-do 13587 (KR); KONG, Taeho, Seongnam-si, Gyeonggi-do 13576 (KR); WON, Jihye, Hwaseong-si, Gyeonggi-do 18466 (KR); KIM, Junsung, Seongnam-si, Gyeonggi-do 13372 (KR); LEE, Soonchul, Seoul 05658 (KR); AN, Borim, Seongnam-si, Gyeonggi-do 13389 (KR)
(74) Representative: Weickmann & Weickmann PartmbB
(86) International application number: PCT/KR2022/005913
(87) International publication number: WO 2022/234998

(57) **Abstract**

According to one aspect, provided are mesenchymal stem cells (MSCs) having enhanced osteogenic differentiation capacity, having excellent osteogenic differentiation capacity, adipogenic differentiation capacity, chondrogenic differentiation capacity, and neurogenic differentiation capacity, and particularly, significantly superior osteogenic differentiation capacity to MSCs derived from other sources. In addition, the MSCs having enhanced osteogenic differentiation capacity exhibit low immunological rejection and have effects of preventing or treating a bone disease and estrogen deficiency syndrome. Furthermore, proliferative ability and karyotype stability of the MSCs having enhanced osteogenic differentiation capacity are maintained even after continuous subculturing, and thus the MSCs having enhanced osteogenic differentiation capacity are safe for commercialization and capable of being mass-produced.

## Description

### Technical Field

This application claims the benefit of Korean Patent Application No. 10-2021-0059516 filed on May 7, 2020, in the Korean Intellectual Property Office, the disclosure of which is incorporated herein in its entirety by reference.

The present disclosure relates to mesenchymal stem cells (MSCs) having enhanced osteogenic differentiation capacity, and use thereof.

### Background Art

Despite fetal mesenchymal stem cells (MSCs) having various advantages in commercialization on the basis of their cellular characteristics, research into fetuses is not actively conducted due to the diversity of numerous cells isolated from rapidly changing stages of fetal development and different anatomical and histological positions, ethical issues about obtaining stem cells from fetuses, and difficulties in obtaining donations of tissue of ectopic or stillborn fetuses for research using ectopic or stillborn fetuses.

Because, as compared to adult stem cells, fetal MSCs (1) express SSEA4, which is a pluripotent stem cell marker, (2) have excellent differentiation capacity, and (3) maintain telomeres of 20 kb or more, the karyotype safety and proliferation rate thereof may be maintained.

In addition, fetal stem cells are known to (4) exhibit lower immunogenicity than MSCs derived from other sources because they secrete immunoregulatory cytokines such as TGF-β and have low expression levels of HLA type I and co-stimulatory molecules.

Therefore, stem cells having enhanced osteogenic differentiation capacity and compositions for preventing or treating bone diseases and/or estrogen deficiency syndrome that mainly occurs after menopause have been developed by using fetal MSCs obtained from donated tissue of ectopic or stillborn fetuses.

### Disclosure

### Technical Problem

Provided are mesenchymal stem cells (MSCs) having enhanced osteogenic differentiation capacity, expressing at least one gene selected from the group consisting of Osx (SP7), c-kit, CD24, CD70, and SFRP2.

Provided also is a pharmaceutical composition for preventing or treating a bone disease including the MSCs having enhanced osteogenic differentiation capacity.

Provided also is a pharmaceutical composition for preventing or treating an estrogen deficiency syndrome including the MSCs having enhanced osteogenic differentiation capacity.

Provided also is a health functional food for preventing or alleviating a bone disease including the MSCs having enhanced osteogenic differentiation capacity.

Provided also is a health functional food for preventing or alleviating an estrogen deficiency syndrome including the MSCs having enhanced osteogenic differentiation capacity.

Provided also is a method of mass-producing MSCs having enhanced osteogenic differentiation capacity, the method including continuously subculturing the MSCs having enhanced osteogenic differentiation capacity.

Provided also is a method of preventing or treating a bone disease, the method including administering the MSCs having enhanced osteogenic differentiation capacity to an individual in need thereof.

Provided also is a method of preventing or treating an estrogen deficiency syndrome, the method including administering the MSCs having enhanced osteogenic differentiation capacity to an individual in need thereof.

### Technical Solution

According to an aspect of the present disclosure, mesenchymal stem cells (MSCs) having enhanced osteogenic differentiation capacity express at least one selected from the group consisting of Osx (SP7), c-kit, CD24, CD70, and SFRP2.

As used herein, the term "mesenchymal stem cell (MSC)" refers to a cell that has self-renewal and sternness maintenance and differentiates into various mesenchymal tissues, and may include MSCs of animals including mammals, e.g., humans.

Also, the term "MSC having enhanced osteogenic differentiation capacity" refers to a MSC having an enhanced function in differentiating into osteocytes, and may be used as a synonym for bone progenitor cell or mesenchymal progenitor cell.

In one aspect, the MSCs having enhanced osteogenic differentiation capacity may be derived from tissue of an ectopic or stillborn fetus, preferably, the tissue of the fetus may be skull tissue of the fetus or calvaria tissue of the fetus positioned at an upper end of the skull, more preferably, the tissue of the fetus may be skull tissue or calvaria tissue excluding neural tubes, even more preferably, calvaria tissue.

Ectopic fetuses may die due to ectopic pregnancy or as a result of surgical abortion after being diagnosed as ectopic pregnancy.

The MSCs having enhanced osteogenic differentiation capacity may express at least one gene selected from the group consisting of Osx (SP7), c-kit, CD24, CD70, and SFRP2, specifically, express all of Osx (SP7), c-kit, CD24, CD70, and SFRP2.

More specifically, the MSCs having enhanced osteogenic differentiation capacity may express Osx (SP7) as a marker specifically expressed in osteogenic MSCs, c-kit as a fetal MSC marker, CD24 and CD70 as cell surface markers, and SFRP2 as an osteogenic differentiation marker by at least about 20 %, 25 %, 30 %, 35 %, 40 %, 45 %, 50 %, 55 %, 60 %, 65 %, 70 %, 75 %, 80 %, 85 %, 90 %, 95 %, 98 %, or about 99 %.

As used herein, the term "positive", with respect to a stem cell marker, may mean that the marker is present in a large amount or a high concentration, as compared to that in other stem cells used as a reference (e.g., umbilical cord Wharton's jelly-derived mesenchymal stem cells (CordSTEMs) and adult bone marrow-derived mesenchymal stem cells (BM-MSCs)). That is, a marker is present inside or on the surface of a cell. Thus, in the case where a cell is distinguished from one or more other cell types by using the marker, the cell is positive for the marker. Also, the positive may mean that the cell has signals with a higher intensity than a background intensity, for example, the cell includes the marker generating the signals in an amount enough to be detectable by a cell measurement device. For example, a cell may be labeled with a CD90-specific antibody to be detectable. In the case where a signal from the antibody is detectably stronger than that of a control (e.g., a background intensity), the cell is "CD90+".

As used herein, the term "negative" means that a marker cannot be detected as compared with a background intensity even by using an antibody specific to a surface marker of a certain cell. For example, in the case where a cell is not detectably labeled with a CD34-specific antibody, the cell is "CD34-".

In addition, in an aspect, the MSCs having enhanced osteogenic differentiation capacity may overexpress at least one gene selected from the group consisting of KITLG, CXCL12, HES4, TWIST1, BMP2, BMPR1B, NOG, and ALPL, as compared to umbilical cord Wharton's jelly-derived MSCs or adult bone marrow-derived MSCs, specifically, may overexpress all of KITLG, CXCL12, HES4, TWIST1, BMP2, BMPR1B, NOG, and ALPL, as compared to umbilical cord Wharton's jelly-derived MSCs or adult bone marrow-derived MSCs.

More specifically, the MSCs having enhanced osteogenic differentiation capacity may overexpress KITLG and CXCL12 as fetal MSC markers and HES4, TWIST1, BMP2, BMPR1B, NOG and ALPL as osteogenic differentiation markers, as compared to umbilical cord Wharton's jelly-derived MSCs or adult bone marrow-derived MSCs.

Cell cultures, lysates, or extracts may be used as alternatives to the MSCs. The cell cultures, lysates, or extracts may be useful alternatives in the case where it is difficult to use cells intact. Due to components of cells such proteins included therein, the cell cultures, lysates, or extracts may exhibit biological activity similar or equivalent to those of original cells. The lysates or extracts may be obtained using commercially available cell lysis kits or extraction kits.

The MSCs having enhanced osteogenic differentiation capacity are safe for commercialization and are easy to mass produce because proliferative ability and karyotype stability may be maintained even after continuous subcultures, as compared to other MSCs (e.g., umbilical cord Wharton's jelly-derived MSCs (CordSTEMs), adult bone marrow-derived MSCs (BM-MSCs) and embryo stem cell-derived MSCs (ES-MSCs). Specifically, the MSCs may have proliferative ability maintained even after 20 passages and karyotype stability maintained even after at least 24 passages.

Also, the MSCs having enhanced osteogenic differentiation capacity according to an aspect have excellent chondrogenic differentiation capacity, adipogenic differentiation capacity, and neurogenic differentiation capacity, particularly, have superior osteogenic differentiation capacity to other MSCs, specifically, superior osteogenic differentiation capacity to that of umbilical cord Wharton's jelly-derived MSCs (CordSTEMs), adult bone marrow-derived MSCs (BM-MSCs), and embryo stem cell-derived MSCs (ES-MSCs).

In an aspect, the MSCs having enhanced osteogenic differentiation capacity may not express HLA class I.

Specifically, in the case where the MSCs having enhanced osteogenic differentiation capacity are cultured in a bFGF-supplemented medium, cells expressing HLA class I may be reduced to 5 % or less. Therefore, the MSCs according to an aspect have a higher cell viability in allogenic cell therapy because fewer cells are killed by immunological rejection.

In addition, in an aspect, the MSCs having enhanced osteogenic differentiation capacity may over-secrete at least one polypeptide selected from the group consisting of ABL1, IGFBP-7, MMP-1, GLO-1, Progranulin, Amylin, BMX, ALPP, FAP, ADAMTS-L2, SPARC, TLR1, Galectin-3, Beta IG-H3, HB-EGF, 11b-HSD1, PTHLP, ACTH, Activin A, GDF11 and GDF3, as compared to umbilical cord Wharton's jelly-derived MSCs or adult bone marrow-derived MSCs, specifically, over-secrete all of ABL1, IGFBP-7, MMP-1, GLO-1, Progranulin, Amylin, BMX, ALPP, FAP, ADAMTS-L2, SPARC, TLR1, Galectin-3, Beta IG-H3, HB-EGF, 11b-HSD1, PTHLP, ACTH, Activin A, GDF11 and GDF3, as compared to umbilical cord Wharton's jelly-derived MSCs or adult bone marrow-derived MSCs.

In addition, the over-secretion may be over-secretion, specifically, into an extracellular culture broth.

The term "polypeptide" refers to a polymer including two or more amino acids linked via amide bonds (or peptide bonds). The polypeptide may include a polypeptide having a sequence homology of about 70 % or more, about 75 % or more, about 80 % or more, about 85 % or more, about 90 % or more, about 92 % or more, about 95 % or more, about 97 % or more, about 98 % or more, or about 99 % or more to an amino acid sequence of the polypeptide.

The term "homology" is used to indicate a degree of relevance to a wild-type amino acid sequence and comparison of homologies may be conducted using a sequence comparison program known in the art. Homology between two or more sequences may be calculated as a percentage (%).

Also, in an aspect, the stem cell may heterogeneously express CD90.

Specifically, the MSCs having enhanced osteogenic differentiation capacity may be derived from bony tissue of a fetus and the tissue of the fetus may be skull or calvaria tissue. In addition, the MSCs may include a cell group with a high expression level of CD90 and a cell group with a low expression level of CD90, and this may be more evident according to addition of bFGF to a culture medium.

According to another aspect, a pharmaceutical composition for preventing or treating a bone disease includes the MSCs having enhanced osteogenic differentiation capacity.

The "MSCs having enhanced osteogenic differentiation capacity" are as described above.

The bone disease may include, for example, at least one selected from the group consisting of osteoporosis, fracture, bone nonunion, osteogenesis imperfecta, osteopenia, osteolytic metastasis, lumbar degenerative kyphosis, Paget disease, bone atrophy, osteomalacia, osteoarthritis, periodontal disease, rickets, aplastic bone disease, bone damage caused by bone metastasis of cancer cells, fibrous dysplasia, McCune Albright syndrome, bone deformity, and osteodysplasia, preferably, at least one selected from the group consisting of osteoporosis, fracture, fibrous dysplasia, McCune Albright syndrome, bone nonunion and osteogenesis imperfecta, more preferably osteoporosis.

The term "prevention" used herein refers to all actions of inhibiting or delaying the onset of a bone disease of an individual by administering a pharmaceutical composition according to an aspect.

The term "treatment" used herein refers to all actions involved in alleviating or beneficially changing symptoms of a bone disease of an individual by administering a pharmaceutical composition according to an aspect.

According to an aspect, the MSCs having enhanced osteogenic differentiation capacity have superior proliferative ability to other MSCs, excellent karyotype stability after proliferation, very high osteogenic differentiation capacity, and remarkable osteogenic ability. Based on the remarkable osteogenic ability, all of bone density, number of osteocytes, and bone volume are increased, and thus remarkable therapeutic effects on a bone disease may be obtained.

Also, in an aspect, the pharmaceutical composition may further include a different pharmaceutical composition for preventing or treating a bone disease in addition to the MSCs having enhanced osteogenic differentiation capacity.

That is, the pharmaceutical composition may be provided in a form mixed with a different composition for preventing or treating a bone disease known in the art or a different composition for preventing or treating a bone disease newly being developed.

In the case where the pharmaceutical composition includes a different composition for preventing or treating a bone disease, it is important to mix a minimum dose capable of obtaining a maximum effect without a side effect and such a dose may be easily determined by those skilled in the art.

In the case where the pharmaceutical composition further includes a different composition for preventing or treating a bone disease, more remarkable synergistic effects of preventing or treating a bone disease are obtained, as compared to a case of including only the MSCs having enhanced osteogenic differentiation capacity as an active ingredient.

In addition, in an aspect, the pharmaceutical composition may be administered alone or in combination with a different composition for preventing or treating a bone disease.

The different composition for preventing or treating a bone disease may be a different composition for preventing or treating a bone disease known in the art or a different composition for preventing or treating a bone disease newly being developed.

The pharmaceutical composition may be administered in combination with the different composition for preventing or treating a bone disease simultaneously, separately, or sequentially in a single dose or multiple doses. It is important to administer a minimum dose capable of obtaining a maximum effect without a side effect in consideration of all of the factors and such a dose may be easily determined by those skilled in the art.

In the case where the pharmaceutical composition is co-administered with a different composition for preventing or treating a bone disease, more remarkable synergistic effects of preventing or treating the bone disease may be obtained in comparison with a case of administering the pharmaceutical composition alone.

The term "administration" refers to introduction of a particular substance into an individual by an appropriate method, and the term "individual" refers to all living creatures such as rats, mice, and livestock including humans. As a specific example, the individual may include mammals including humans.

In an aspect, administration routes of the pharmaceutical composition include, but are not limited to, oral, intravenous, intramuscular, intraarterial, periosteal or intramedullary, intrathecal, intracardiac, transdermal, subcutaneous, intraperitoneal, intranasal, enteral, topical, or sublingual routes.

The pharmaceutical composition is administered in a pharmaceutically effective amount. The term "pharmaceutically effective amount" refers to an amount sufficient for treating a disease at a reasonable benefit/risk ratio applicable to medical treatment, and a level of an effective dose may be determined based on factors including age, gender, status, and body weight of a patient, absorption rate of active ingredients in vivo, inactivation rate, excretion speed, type of disease, severity of disease, drug activity, drug sensitivity, administration time, administration route, excretion rate, treatment duration, severity of obesity, and drug(s) to be concurrently used in combination, and other factors well-known in the medical field. For example, the pharmaceutical composition may be administered at a dose of 0.001 mg/kg/day to 1000 mg/kg/day, and may be administered at a dose of 10² cells/kg to 10⁹ cells/kg, 10³ cells/kg to 10⁹ cells/kg, 10³ cells/kg to 10⁸ cells/kg, 10⁴ cells/kg to 10⁸ cells/kg, or 10⁴ cells/kg to 10⁷ cells/kg based on the MSCs having enhanced osteogenic differentiation capacity contained in the pharmaceutical composition and the individual.

According to another aspect, a pharmaceutical composition for preventing or treating an estrogen deficiency syndrome includes the MSCs having enhanced osteogenic differentiation capacity.

The "MSCs having enhanced osteogenic differentiation capacity" are as described above.

The estrogen deficiency syndrome may exhibit at least one symptom selected from the group consisting of facial blushing, perspiration, insomnia, nervousness, depression, dizziness, concentration disorder, short-term memory impairment, anxiety, memory loss, palpitation, muscle pain, joint pain, skin dryness and atrophy, vaginal dryness, vaginal atrophy, lower urethral atrophy, vaginitis, uterine atrophy, cystitis, pain in urination, urinary urgency, obesity, type II diabetes, hyperlipidemia, arteriosclerosis, fatty liver, sudden heart rate change, sleep disorder, loss of confidence and sexual desire, osteoporosis, atherosclerotic heart disease, venous thrombosis, irregular menstrual cycle, irregular menstrual flow, irregular menstrual period, hyperhidrosis, tinnitus, hypertension, digestive disorder, headache, cognitive dysfunction, fatigue, mood swing, Alzheimer's disease, dyspareunia, short-term memory disorder, decreased libido, blood flow disorder, and skin aging which are caused by estrogen deficiency, preferably exhibit at least one symptom selected from the group consisting of obesity and type II diabetes caused by estrogen deficiency, more preferably exhibit osteoporosis and obesity caused by estrogen deficiency.

The term "prevention" used herein refers to all actions of inhibiting or delaying the onset of an estrogen deficiency syndrome of an individual by administering a pharmaceutical composition according to an aspect.

The term "treatment" used herein refers to all actions involved in alleviating or beneficially changing symptoms of an estrogen deficiency syndrome of an individual by administering a pharmaceutical composition according to an aspect.

According to an aspect, the MSCs having enhanced osteogenic differentiation capacity have superior proliferative ability to other MSCs, excellent karyotype stability after proliferation, and very high osteogenic differentiation capacity, and inhibits an increase in adipocytes. Therefore, the MSCs having enhanced osteogenic differentiation capacity may exhibit effects of preventing or treating an estrogen deficiency syndrome.

In addition, according to an aspect, the pharmaceutical composition may further include a different pharmaceutical composition for preventing or treating an estrogen deficiency syndrome in addition to the MSCs having enhanced osteogenic differentiation capacity.

That is, the pharmaceutical composition may be provided in a form mixed with a different composition for preventing or treating an estrogen deficiency syndrome known in the art or a different composition for preventing or treating an estrogen deficiency syndrome newly being developed.

In the case where the pharmaceutical composition includes a different composition for preventing or treating an estrogen deficiency syndrome, it is important to mix a minimum dose capable of obtaining a maximum effect without a side effect and such a dose may be easily determined by those skilled in the art.

In the case where the pharmaceutical composition further increases a different composition for preventing or treating an estrogen deficiency syndrome, more remarkable synergistic effects of preventing or treating the estrogen deficiency syndrome may be obtained in comparison with a case of including the MSCs having enhanced osteogenic differentiation capacity as an active ingredient.

In addition, in an aspect, the pharmaceutical composition may be administered alone or in combination with a different composition for preventing or treating an estrogen deficiency syndrome.

The different composition for preventing or treating an estrogen deficiency syndrome may be a different composition for preventing or treating an estrogen deficiency syndrome known in the art or a different composition for preventing or treating an estrogen deficiency syndrome newly being developed.

The pharmaceutical composition may be administered in combination with the different composition for preventing or treating an estrogen deficiency syndrome simultaneously, separately, or sequentially in a single dose or multiple doses. It is important to administer a minimum dose capable of obtaining a maximum effect without a side effect in consideration of all of the factors and such a dose may be easily determined by those skilled in the art.

In the case where the pharmaceutical composition is co-administered with a different composition for preventing or treating an estrogen deficiency syndrome, more remarkable synergistic effects of preventing or treating the estrogen deficiency syndrome may be obtained in comparison with a case of administering the pharmaceutical composition alone.

The term "administration" refers to introduction of a particular substance into an individual by an appropriate method, and the term "individual" refers to all living creatures such as rats, mice, and livestock including humans. As a specific example, the individual may include mammals including humans.

The pharmaceutical compositions may include an active ingredient alone or may further include at least one pharmaceutically acceptable carrier, excipient, or diluent.

Specifically, the carrier may be a colloidal suspension, a powder, a saline solution, a lipid, a liposome, a microsphere, or a nano-spherical particle. They may form a complex or associated with a delivery vehicle and may be delivered in vivo by using any delivery system known in the art such as a lipid, a liposome, a microparticle, gold, a nanoparticle, a polymer, a condensed agent, a polysaccharide, a polyamino acid, a dendrimer, saponin, an adsorption enhancer, or a fatty acid.

The pharmaceutical compositions may be formulated by using a diluent or excipient commonly used in the art such as a lubricant, a sweetener, a flavoring agent, an emulsifier, a suspension, a preservative, a filler, an extender, a binder, a humectant, a disintegrant, and a surfactant. Solid formulations for oral administration may include tablets, pills, powders, granules, capsules, etc. The solid formulations may be prepared by adding at least one excipient such as starch, calcium carbonate, sucrose or lactose, and gelatin. In addition, a lubricant such as magnesium stearate and talc may be used in addition to simple excipients. Liquid preparations for oral dosage include suspensions, liquids for internal use, emulsions, syrups, and the like. In addition to a simple diluent such as water or liquid paraffin, various excipients such as a humectant, a sweetener, a flavoring agent, and a preservative may be contained in the liquid preparations. Preparations for parenteral dosage include a sterile aqueous solution, a non-aqueous solvent, a suspension, an emulsion, a lyophilizate, a suppository, and the like. Propylene glycol, polyethylene glycol, vegetable oils such as olive oil, and injectable esters such as ethyl oleate may be suitable for the non-aqueous solvent and suspension. As a base for suppositories, witepsol, macrogol, tween 61, cacao butter, laurin butter, glycerogelatin, and the like may be used, and a known diluent, excipient, or the like in the form of an eye drop may be used in the manufacture.

According to an aspect, administration routes of the pharmaceutical compositions include, but are not limited to, oral, intravenous, intramuscular, intraarterial, intramedullary, intrathecal, intracardiac, transdermal, subcutaneous, intraperitoneal, intranasal, enteral, topical, or sublingual routes.

The pharmaceutical compositions are administered in a pharmaceutically effective amount. The term "pharmaceutically effective amount" refers to an amount sufficient for treating a disease at a reasonable benefit/risk ratio applicable to medical treatment, and a level of an effective dose may be determined based on factors including age, gender, status, and body weight of a patient, absorption rate of active ingredients in vivo, inactivation rate, excretion speed, type of disease, severity of disease, drug activity, drug sensitivity, administration time, administration route, excretion rate, treatment duration, severity of obesity, and drug(s) to be concurrently used in combination, and other factors well-known in the medical field. For example, the pharmaceutical compositions may be administered at a dose of 0.001 mg/kg/day to 1000 mg/kg/day, and may be administered at a dose of 10² cells/kg to 10⁹ cells/kg, 10³ cells/kg to 10⁹ cells/kg, 10³ cells/kg to 10⁸ cells/kg, 10⁴ cells/kg to 10⁸ cells/kg, or 10⁴ cells/kg to 10⁷ cells/kg based on the MSCs having enhanced osteogenic differentiation capacity contained in the pharmaceutical composition and the individual.

According to another aspect, a health functional food for preventing or alleviating a bone disease includes the MSCs having enhanced osteogenic differentiation capacity.

The "MSCs having enhanced osteogenic differentiation capacity", "bone disease", "prevention", and the like are as described above.

The term "alleviation", refers to all actions that at least reduce parameters related to a condition to be treated, for example, a degree of a symptom. In this case, the health functional food may be used to prevent or alleviate a bone disease simultaneously in combination with or separately from a drug for treatment, before or after the occurrence of the disease.

In an aspect, the health functional food may further include a different health functional food for preventing or alleviating a bone disease.

The health functional food may be provided in a form mixed with a different health functional food for preventing or alleviating a bone disease known in the art or a different health functional food for preventing or alleviating a bone disease newly being developed.

In the case where the health functional food includes a different health functional food for preventing or alleviating a bone disease, it is important to mix an amount capable of obtaining a maximum effect without a side effect and such a dose may be easily determined by those skilled in the art.

In the case where the health functional food further includes a different health functional food for preventing or alleviating a bone disease, more remarkable synergistic effects of preventing or alleviating a bone disease may be obtained, as compared to a case including only the MSCs having enhanced osteogenic differentiation capacity as an active ingredient.

The health functional food may be taken in combination with the different health functional food for preventing or alleviating a bone disease known in the art or the different health functional food for preventing or alleviating a bone disease newly being developed simultaneously, separately, or sequentially in a single dose or multiple doses. It is important to take a minimum amount capable of obtaining a maximum effect without a side effect in consideration of all of the factors and such an amount may be easily determined by those skilled in the art.

In the case where the health functional food is taken in combination with a different health functional food for preventing or alleviating a bone disease, more remarkable synergistic effects of preventing or alleviating the bone disease may be obtained in comparison with a case of taking the health functional food alone.

According to another aspect, a health functional food for preventing or alleviating an estrogen deficiency syndrome includes the MSCs having enhanced osteogenic differentiation capacity.

The "MSCs having enhanced osteogenic differentiation capacity", "estrogen deficiency syndrome", and "prevention" are as described above.

The term "alleviation", refers to all actions that at least reduce parameters related to a condition to be treated, for example, a degree of a symptom. In this case, the health functional food may be used to prevent or alleviate an estrogen deficiency syndrome simultaneously in combination with or separately from a drug for treatment, before or after the occurrence of the disease.

In an aspect, the health functional food may further include a different health functional food for preventing or alleviating an estrogen deficiency syndrome.

The health functional food may be provided in a form mixed with a different health functional food for preventing or alleviating an estrogen deficiency syndrome known in the art or a different health functional food for preventing or alleviating an estrogen deficiency syndrome newly being developed.

In the case where the health functional food includes a different health functional food for preventing or alleviating an estrogen deficiency syndrome, it is important to mix a minimum amount capable of obtaining a maximum effect without a side effect and such a dose may be easily determined by those skilled in the art.

In the case where the health functional food further includes a different health functional food for preventing or alleviating an estrogen deficiency syndrome, more remarkable synergistic effects of preventing or alleviating an estrogen deficiency syndrome may be obtained, as compared to a case including only the MSCs having enhanced osteogenic differentiation capacity as an active ingredient.

The health functional food may be taken in combination with the different health functional food for preventing or alleviating an estrogen deficiency syndrome known in the art or the different health functional food for preventing or alleviating an estrogen deficiency syndrome newly being developed simultaneously, separately, or sequentially in a single dose or multiple doses. It is important to take a minimum amount capable of obtaining a maximum effect without a side effect in consideration of all of the factors and such an amount may be easily determined by those skilled in the art.

In the case where the health functional food is taken in combination with a different health functional food for preventing or alleviating an estrogen deficiency syndrome, more remarkable synergistic effects of preventing or alleviating the estrogen deficiency syndrome may be obtained in comparison with a case of taking the health functional food alone.

In the health functional foods, the active ingredient may be directly added to the foods or may be used together with other foods or food ingredients and may be appropriately used according to any methods known in the art. A mixing amount of the active ingredient may be appropriately determined according to the purpose of use (prevention or alleviation). In general, the health functional foods may be added in an amount of about 15 wt% or less, more specifically, about 10 wt% or less based on an raw ingredients while manufacturing of foods or drinks. However, in a case of a long-term intake of the food for the purpose of health and hygiene or for health control, the amount may be less than the above range.

The health functional foods may be formulated into one selected from the group consisting of tablets, pills, powders, granules, capsules, and liquids, by including at least one of a carrier, a diluent, an excipient, and an additive. Foods to which the MSCs may be added are various types of foods, powders, granules, tablets, capsules, syrups, beverages, gums, teas, vitamin complexes, and health aid foods.

Examples of the carrier, excipient, diluent, and additive may include at least one selected from the group consisting of lactose, dextrose, sucrose, sorbitol, mannitol, erythritol, starch, acacia rubber, calcium phosphate, alginate, gelatin, calcium phosphate, calcium silicate, micro-crystalline cellulose, polyvinyl pyrrolidone, cellulose, polyvinyl pyrrolidone, methyl cellulose, water, sugar syrup, methyl cellulose, methylhydroxy benzoate, propylhydroxy benzoate, talc, magnesium stearate, and mineral oil.

The health functional foods may include other ingredients, except for the active ingredient described above, as essential ingredients without particular limitation. For example, various types of flavoring agents or natural carbohydrates, and the like may be contained as additional ingredients as in the conventional beverages. Examples of the natural carbohydrates may include common saccharides such as monosaccharides such as glucose and fructose, disaccharides such as maltose and sucrose, and polysaccharides such as dextrin and cyclodextrin; and sugar alcohols such as xylitol, sorbitol, and erythritol. As flavoring agents other than those described above, natural flavoring agents (such as taumatin and stevia extracts (e.g., rebaudioside A and glycyrrhizin)) and synthetic flavoring agents (such as saccharin and aspartame) may be advantageously used. A percentage of the natural carbohydrates may be appropriately determined by those skilled in the art.

Additionally, the health functional foods according to an aspect may include a variety of nutrients, vitamins, minerals (electrolytes), synthetic and natural flavoring agents, colorants and fillers (cheese, chocolate, etc.), pectic acid or salts thereof, alginic acid or salts thereof, organic acids, protective colloidal thickening agents, pH modifiers, stabilizers, preservatives, glycerin, alcohols, carbonating agents used in carbonated beverages, etc. These ingredients may be used independently or in combination. The percentage of these additives may also be appropriately selected by those skilled in the art.

According to another aspect of the present disclosure, a method of mass-producing MSCs having enhanced osteogenic differentiation capacity includes continuously subculturing the MSCs having enhanced osteogenic differentiation capacity.

The "MSCs having enhanced osteogenic differentiation capacity" may be as described above.

The "subculture" refers to producing new cells or microorganisms by transferring some cells from an original culture to a fresh culture medium and culturing the same. The number of cells or microorganisms may be increased or lifespan of cell lines may be extended by subculturing.

The "continuously subculturing" refers subculturing without changing innate characteristics of original cells or microorganisms.

In the case of the MSCs having enhanced osteogenic differentiation capacity, unlike general MSCs, innate characteristics, i.e., osteogenic differentiation capacity, adipogenic differentiation capacity, chondrogenic differentiation capacity, neurogenic differentiation capacity, low immunological rejection, and effects of preventing or treating a bone disease and/or an estrogen deficiency syndrome, are not deteriorated, and karyotype stability thereof may be maintained even after continuous subculturing.

According to the mass-producing method according to an aspect, the MSCs having enhanced osteogenic differentiation capacity may be proliferated in large quantities, and thus the MSCs having enhanced osteogenic differentiation capacity may be commercialized.

According to another aspect of the present disclosure, a method of preventing or treating an estrogen deficiency syndrome includes administering the MSCs having enhanced osteogenic differentiation capacity to an individual in need thereof.

The "MSCs having enhanced osteogenic differentiation capacity", "individual", "administration", "estrogen deficiency syndrome", "prevention", and "treatment" are as described above.

According to another aspect, provided is a use of the MSCs having enhanced osteogenic differentiation capacity for preventing or treating a bone disease.

The "MSCs having enhanced osteogenic differentiation capacity", "bone disease", " prevention", and "treatment" are as described above.

According to another aspect, provided is a use of the MSCs having enhanced osteogenic differentiation capacity for preventing or treating an estrogen deficiency syndrome.

The "MSCs having enhanced osteogenic differentiation capacity", "estrogen deficiency syndrome", "prevention", and "treatment" are as described above.

### Advantageous Effects

The MSCs having enhanced osteogenic differentiation capacity according to an aspect have excellent osteogenic differentiation capacity, adipogenic differentiation capacity, chondrogenic differentiation capacity, and neurogenic differentiation capacity, and particularly, significantly superior osteogenic differentiation capacity to that of MSCs derived from other sources. In addition, the MSCs having enhanced osteogenic differentiation capacity have low immunological rejection and effects of preventing or treating a bone disease and an estrogen deficiency syndrome. Furthermore, the MSCs having enhanced osteogenic differentiation capacity have proliferative ability and karyotype stability maintained even after continuous subculturing, and thus it is advantageous that the MSCs are safe for commercialization and capable of being mass-produced.

### Description of Drawings

FIGS. 1 and 2 show proliferation of fetal mesenchymal stem cell (fetal MSC)-derived MSCs having enhanced osteogenic differentiation capacity. Specifically, FIG. 1 shows shapes of fetal MSC-derived MSCs having enhanced osteogenic differentiation capacity, and FIG. 2 shows growth curves and expected numbers of cells of fetal MSC-derived MSCs having enhanced osteogenic differentiation capacity.
FIG. 3 shows differentiation capacity of fetal MSC-derived MSCs having enhanced osteogenic differentiation capacity. Specifically, A shows osteogenic differentiation of fetal MSC-derived MSCs having enhanced osteogenic differentiation capacity, B shows adipogenic differentiation of fetal MSC-derived MSCs having enhanced osteogenic differentiation capacity, C shows chondrogenic differentiation of fetal MSC-derived MSCs having enhanced osteogenic differentiation capacity, and D shows neurogenic differentiation of fetal MSC-derived MSCs having enhanced osteogenic differentiation capacity.
FIG. 4 shows results of comparison of differentiation capacity between fetal skull tissue-derived MSCs having enhanced osteogenic differentiation capacity and limb tissue-derived MSCs having enhanced osteogenic differentiation capacity.
FIGS. 5 to 7 show results of comparison of osteogenic differentiation capacity. Specifically, FIG. 5 shows osteogenic differentiation of MSCs derived from various sources, FIG. 6 shows results of comparison of osteogenic differentiation capacity between fetal MSC-derived MSCs having enhanced osteogenic differentiation capacity and bone marrow-mesenchymal stem cells (BM-MSCs), and FIG. 7 shows gene expression of osteogenic markers during osteogenic differentiation of fetal MSC-derived MSCs having enhanced osteogenic differentiation capacity.
FIG. 8 shows karyotypes of fetal MSC-derived MSCs having enhanced osteogenic differentiation capacity.
FIGS. 9 to 12 show the expression of surface markers of fetal MSC-derived MSCs having enhanced osteogenic differentiation capacity by FACS analysis. Specifically, FIG. 9 shows results of FACS analysis of MSC markers, FIG. 10 shows results of FACS analysis of totipotent stem cell markers, FIG. 11 shows results of FACS analysis of hematopoietic stem cell markers, and FIG. 12 shows results of FACS analysis of human leukocyte antigens.
FIGS. 13 to 16 show results of RNA sequencing analysis of MSCs derived from various sources. Specifically, FIG. 13 shows a clustering heat map and a principal component analysis, FIG. 14 shows analysis of differentially expressed genes, and FIGS. 15 and 16 show the expression of mRNA.
FIG. 17 shows secretome analysis results, specifically, a clustering heat map and principal component analysis results.
FIGS. 18A to 18C show scatter plots for secretome analysis. FIG. 18A shows results of comparison between fetal MSCs (+) and adult bone marrow-derived MSCs, FIG. 18B shows results of comparison between fetal MSCs (+) and umbilical cord Wharton's jelly-derived MSCs (CordSTEMs), and FIG. 18C shows results of comparison between fetal MSCs (+) and fetal MSCs (-).
FIG. 19 shows heterogeneity of CD90 identified by FACS analysis. Specifically, FIG. 19 shows FACS analysis results.
FIGS. 20A to 20C show heterogeneity of CD90 identified by FACS analysis. Specifically, FIGS. 20A to 20C show FACS analysis results and osteogenic differentiation levels of bony tissue, skin tissue, and limb tissue of fetuses.
FIG. 21 shows a plan of an experiment to identify the ability of fetal MSC-derived MSCs having enhanced osteogenic differentiation capacity to protect bones.
FIGS. 22A and 22B show bone protection by fetal MSC-derived MSCs having enhanced osteogenic differentiation capacity. Specifically, FIG. 22A shows images obtained by micro-computerized tomography, and FIGS. 22B shows results of calculating bone volume, trabecular bone number, and bond density from the images of FIG. 22A.
FIGS. 23 to 24 show estrogen deficiency syndrome protection by fetal MSC-derived MSCs having enhanced osteogenic differentiation capacity. FIG. 23 shows a plan of an experiment, and FIG. 24 shows results of analysis of fatty tissue.

### Best Mode

Hereinafter, the present disclosure will be described in more detail with reference to the following examples. However, these examples are for illustrative purposes only, and the scope of the disclosure is not limited by these examples.

### Examples

### Example 1. Proliferation of Fetal Mesenchymal Stem Cell (Fetal MSC)-derived MSCs having Enhanced Osteogenic Differentiation Capacity in the Presence and Absence of Basic Fibroblast Growth Factor (bFGF) Additive

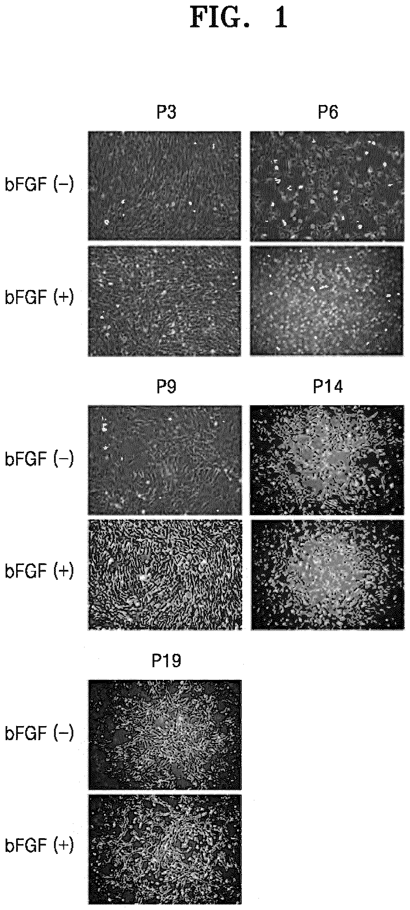

Proliferation of fetal MSC-derived MSCs having enhanced osteogenic differentiation capacity in the presence and absence of a basic fibroblast growth factor (bFGF) additive was identified, and the fetal MSC-derived MSCs having enhanced osteogenic differentiation capacity was expanded into a culture medium in the presence (lower portion of FIG. 1) and absence (upper portion of FIG. 1) of the bFGF additive. Fetal MSCs were subcultured and the number of cells was counted every 3 to 4 days as instructed. As a result, as shown in FIG. 1, shapes of the fetal MSC-derived MSCs having enhanced osteogenic differentiation capacity were identified.

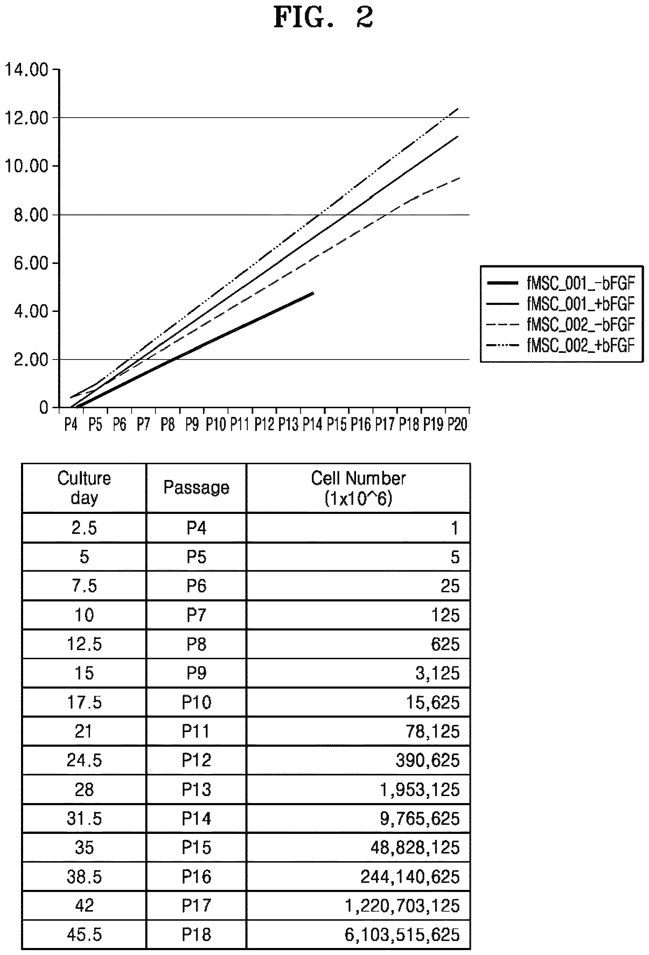

In addition, two other fetal MSC-derived mesenchymal stem cell lines having enhanced osteogenic differentiation capacity (fMSC_001 and fMSC_002) were identified and growth curves (upper portion of FIG. 2) and expected numbers of cells (lower portion of FIG. 2) of the fetal MSC-derived MSCs having enhanced osteogenic differentiation capacity are shown in FIG. 2.

Referring to FIGS. 1 and 2, it was confirmed that the fetal MSC-derived MSCs having enhanced osteogenic differentiation capacity had spindle morphology that is a morphological property of MSCs regardless of addition of bFGF.

However, due to superior proliferative ability to that of adult-derived MSC (BM-MSCs and AD-MSCs) and neonatal MSCs (umbilical cord MSCs, placental MSCs, and UC blood MSCs), it was confirmed that the proliferative ability of the MSCs was maintained up to 20 passages.

In terms of mass production, this indicates that 6 billion vials or more may be produced based on 10⁶ cells after 18 passages.

### Example 2. Differentiation Capacity of Fetal MSC-derived MSCs Having Enhanced Osteogenic Differentiation Capacity

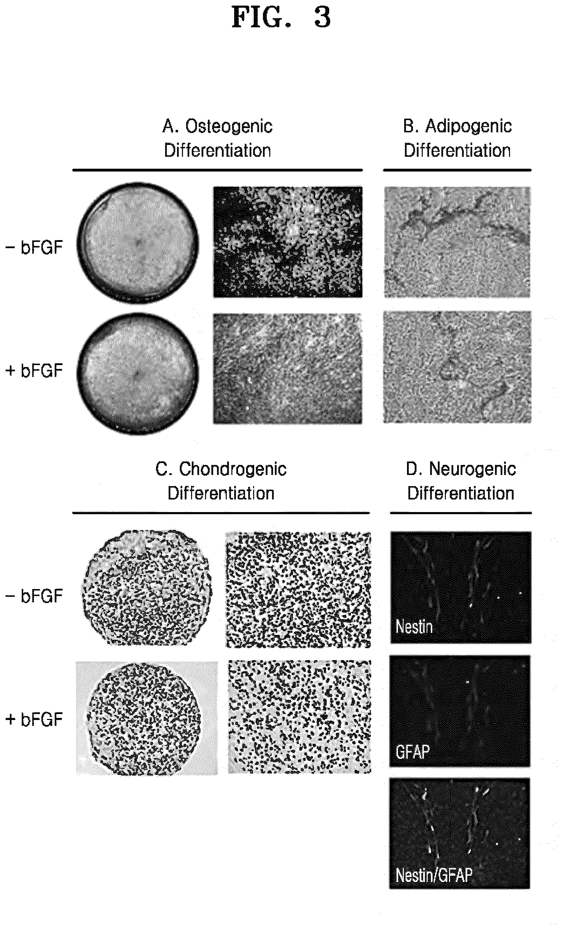

Differentiation capacity of the fetal MSC-derived MSCs having enhanced osteogenic differentiation capacity was evaluated in the presence and absence of the bFGF by identifying osteogenic differentiation capacity (FIG. 3A), adipogenic differentiation capacity (FIG. 3B), chondrogenic differentiation capacity (FIG. 3C), and neurogenic differentiation capacity (FIG. 3D).

Specifically, fetal MSC-derived MSCs having enhanced osteogenic differentiation capacity were expanded into a culture medium in the presence of bFGF (lower portion) and in the absence thereof (upper portion).

In order to identify osteogenic differentiation capacity, fetal MSC-derived MSCs having enhanced osteogenic differentiation capacity were cultured in an osteogenic differentiation medium (StemPro^{®} Osteogenesis Differentiation Kit) for 14 days. After differentiation, extracellular calcium deposits were stained with Alizarin Red (FIG. 3A).

In addition, in order to identify adipogenic differentiation capacity, fetal MSC-derived MSCs having enhanced osteogenic differentiation capacity were culture in an adipogenic differentiation medium (StemPro^{®} Adipogenesis Differentiation Kit) for 10 days. After differentiation, oil drops were stained with an Oil Red solution (FIG. 3B).

In addition, in order to identify chondrogenic differentiation capacity, cell pellets (2.5 ×10⁵ cells/pellet) were cultured in a chondrogenic differentiation medium (DMEM/high-glucose supplemented with 1 % FBS, 1X ITS+Premix, 10 ng/mL TGF-β1, 10 µM dexamethasone, 1 µM ascorbate-2-phosphate, 1 % sodium pyruvate, 1X NEAA and 1 % penicillin/streptomycin) for 21 days. After differentiation, paraffin sections were stained with H&E (FIG. 3C).

In addition, in order to identify neurogenic differentiation capacity, fetal MSC-derived MSCs having enhanced osteogenic differentiation capacity were cultured in a neurogenic differentiation medium supplemented with B-27 serum-free supplement and GlutaMAX-I for 14 days. After differentiation, differentiated neurons were stained by an immunocytochemistry method using Nestin or GFAP-specific antibodies.

As a result, it was confirmed that, fetal MSC-derived MSCs having enhanced osteogenic differentiation capacity had differentiation characteristics of MSCs, such as osteogenic differentiation capacity, adipogenic differentiation capacity, chondrogenic differentiation capacity, and slight neurogenic differentiation capacity, maintained regardless of addition of bFGF.

### Example 3. Comparison of Differentiation Capacity between MSCs Having Enhanced Osteogenic Differentiation Capacity Derived from Skull or Calvaria Tissue of Ectopic or Stillborn Human Fetus and Limb Tissue-derived MSCs Having Enhanced Osteogenic Differentiation Capacity

Differentiation capacity of MSCs having enhanced osteogenic differentiation capacity derived from skull or calvaria tissue of ectopic or stillborn human fetuses was compared with that of MSCs having enhanced osteogenic differentiation capacity derived from limbs of ectopic or stillborn human fetuses.

Specifically, in order to identify osteogenic differentiation capacity, fetal MSC (limb tissue)-derived MSCs having enhanced osteogenic differentiation capacity were cultured in an osteogenic differentiation medium (StemPro^{®} Osteogenesis Differentiation Kit) for 14 days. After differentiation, extracellular calcium deposits were stained with Alizarin Red.

In addition, in order to identify adipogenic differentiation capacity, fetal MSC (limb tissue)-derived MSCs having enhanced osteogenic differentiation capacity were cultured in an adipogenic differentiation medium (StemPro^{®} Adipogenesis Differentiation Kit) for 10 days. After differentiation, oil drops were stained with Oil Red.

In addition, in order to identify chondrogenic differentiation capacity, cell pellets (2.5 ×10⁵ cells/pellet) were cultured in a chondrogenic differentiation medium (DMEM/high-glucose supplemented with 1 % FBS, 1X ITS+Premix, 10 ng/mL TGF-β1, 10 µM dexamethasone, 1 µM ascorbate-2-phosphate, 1 % sodium pyruvate, 1X NEAA and 1 % penicillin/streptomycin) for 21 days. After differentiation, paraffin sections were stained with H&E.

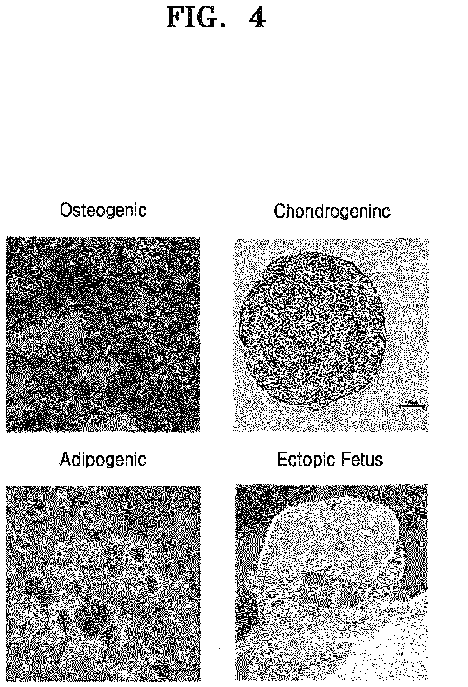

As a result, it was confirmed that the fetal MSC-derived MSCs having enhanced osteogenic differentiation capacity isolated form limbs of fetuses had similar adipogenic differentiation capacity, slightly increased chondrogenic differentiation capacity, and slightly decreased osteogenic differentiation capacity, as compared to those of fetal MSC-derived MSCs having enhanced osteogenic differentiation capacity isolated from skull or calvaria tissue of fetuses (FIG. 4).

Fetal MSC-derived MSCs having enhanced osteogenic differentiation capacity isolated form skull or calvaria tissue of fetuses were used in all examples other than Example 3.

### Example 4. Comparison of Osteogenic Differentiation Capacity

Osteogenic differentiation capacities of MSCs derived from various sources were compared.

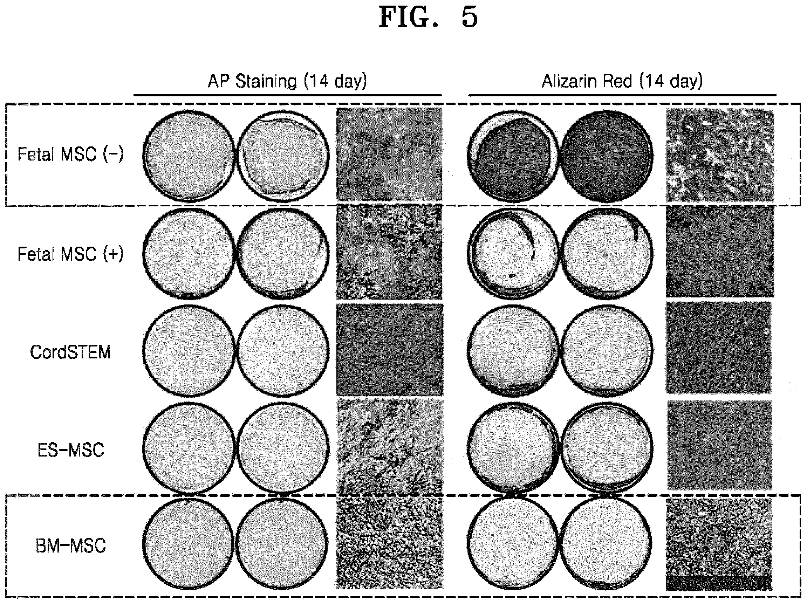

Specifically, MSCs including umbilical cord Wharton's jelly-derived MSCs (CordSTEMs), adult bone marrow-derived MSCs (BM-MSCs) and embryo stem cell-derived MSCs (ES-MSCs) were expanded into culture media. MSCs were cultured in an osteogenic differentiation medium (StemPro^{®} Osteogenesis Differentiation Kit) for 14 days. After differentiation, extracellular calcium deposits and enzymatic activity of alkaline phosphatase were identified by staining respectively with Alizarin Red and AP (FIG. 5).

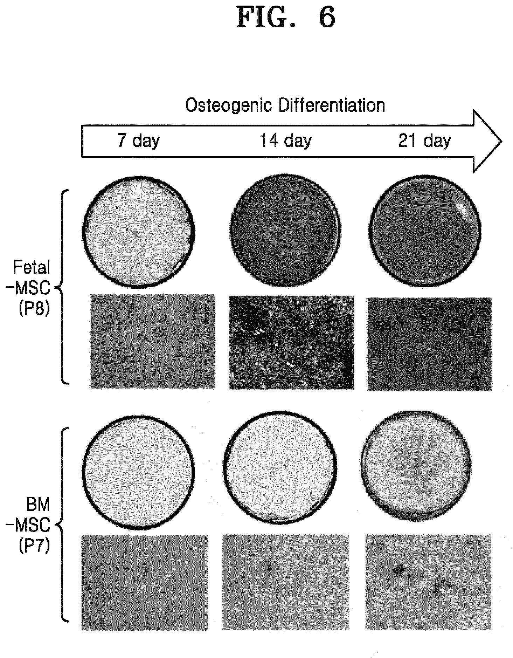

In addition, for comparison of osteogenic differentiation capacity between fetal MSC-derived MSCs having enhanced osteogenic differentiation capacity and BM-MSCs, the MSCs were cultured in an osteogenic differentiation medium (StemPro^{®}Osteogenesis Differentiation Kit) for indicated periods of time (7, 14, and 21 days). After differentiation, extracellular calcium deposits were stained with Alizarin Red (FIG. 6).

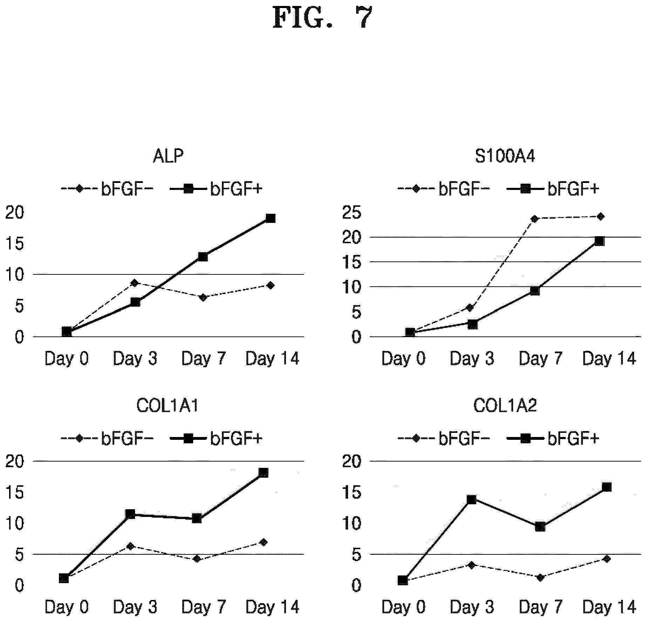

In addition, in order to identify expression of osteogenic marker genes, total RNA was prepared as shown during osteogenic differentiation, and RT-PCR was performed using primers for the osteogenic marker genes (FIG. 7) (alkaline phosphatase (ALP), S100 calcium binding protein A4 (S100A4), collagen type I alpha 1 chain (COL1A1), and collagen type I alpha 2 chain (COL1A2)).

As a result, based on comparison analysis of osteogenic differentiation capacity, it was confirmed that the fetal MSC-derived MSCs having enhanced osteogenic differentiation capacity, particularly in the case of being cultured in a bFGF-free medium, had superior osteogenic differentiation capacity to those of adult-derived MSCs (BM-MSC), neonatal MSCs (Umbilical Cord MSC), and embryo stem cell-derived MSCs (ES-MSCs) (FIG. 5).

In comparison with BM-MSCs, which are known to have the highest osteogenic differentiation capacity, the fetal MSC-derived MSCs having enhanced osteogenic differentiation capacity exhibited osteogenic differentiation initiated at 7 days and saturated at 14 days after osteogenic differentiation was initiated, while the osteogenic differentiation of the MB-MSCs was not saturated even after 21 days. Also, this result was confirmed by expression of various other osteogenic differentiation marker genes.

### Example 5. Karyotyping

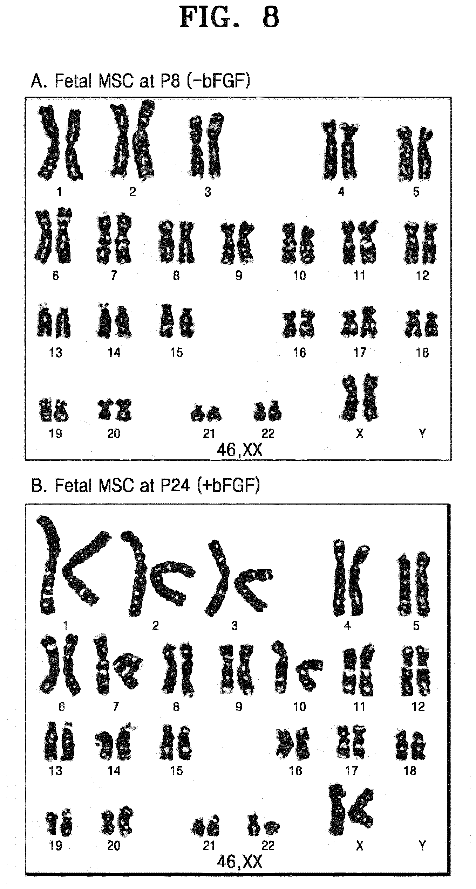

To perform karyotyping of fetal MSC-derived MSCs having enhanced osteogenic differentiation capacity, the fetal MSC-derived MSCs having enhanced osteogenic differentiation capacity were expanded into a culture medium in the presence or absence of a bFGF additive. The fetal MSC-derived MSCs having enhanced osteogenic differentiation capacity were cultured up to passage 8 in the case of not adding bFGF (FIG. 8A) and up to passage 24 in the case of adding bFGF (FIG. 8B) as shown. At least 10 cells were analyzed and shown as representative G (Giemsa) band metaphase microscopic images.

As a result of identify karyotype stability based on karyotyping, karyotype stability was maintained even at passages 8 and 24, and thus it was confirmed that the fetal MSC-derived MSCs having enhanced osteogenic differentiation capacity are safe for commercialization.

### Example 6. Expression of Surface Marker

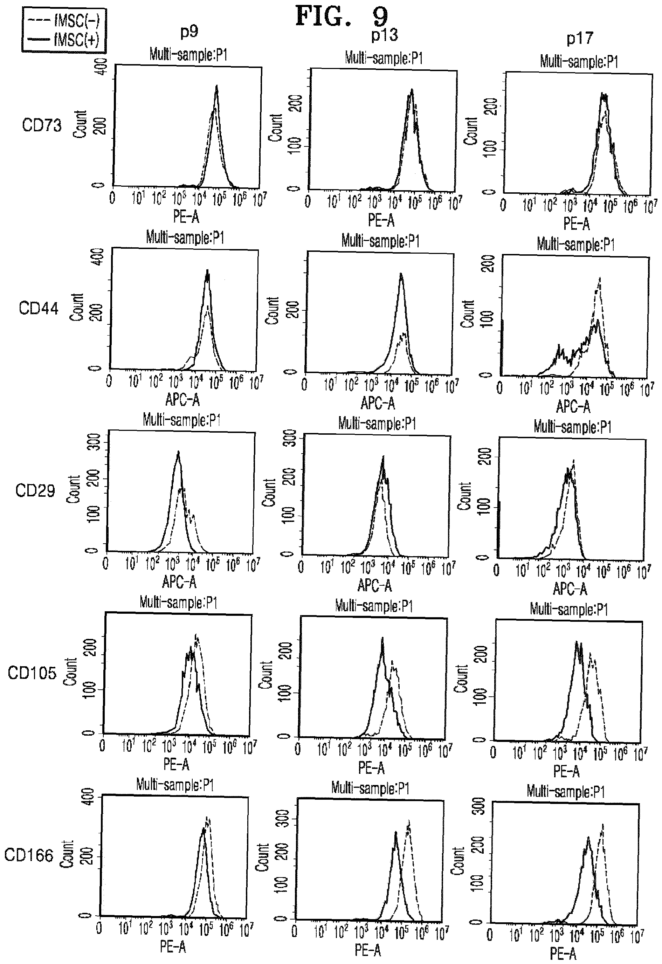

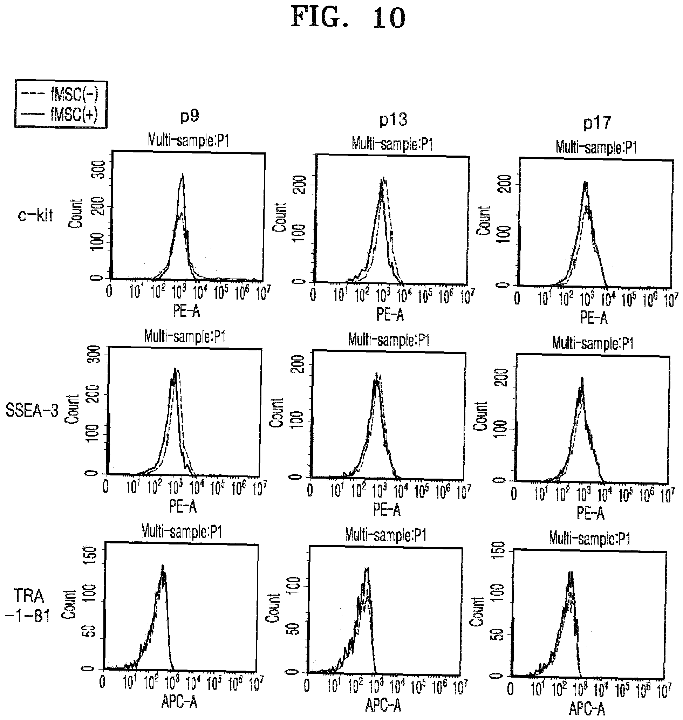

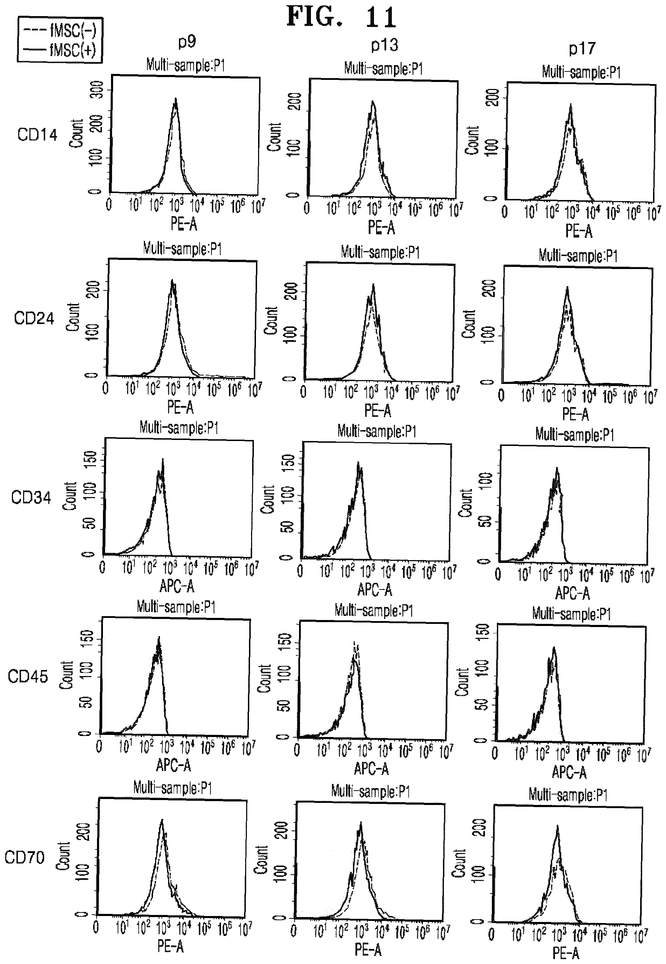

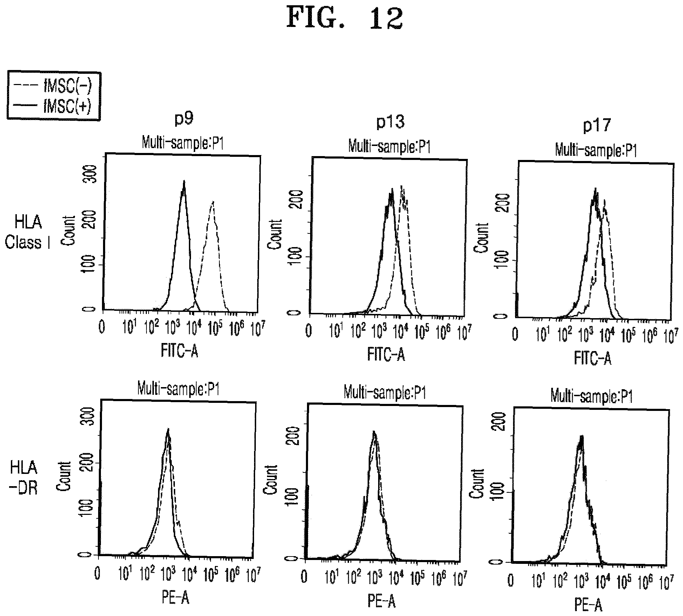

Expression of surface markers of fetal MSC-derived MSCs having enhanced osteogenic differentiation capacity was identified by FACS analysis. The fetal MSC-derived MSCs having enhanced osteogenic differentiation capacity was expanded to a culture medium in the presence or absence of bFGF. The cells were cultured in the shown passages, and FACS analysis was performed by using MSC markers (FIG. 9), totipotent stem cell markers (FIG. 10), hematopoietic stem cell markers (FIG. 11), and human leukocyte antigens (FIG. 12).

As a result of identifying expression of surface markers of MSCs by FACS analysis in order to identify MSC characteristics of the fetal MSC-derived MSCs having enhanced osteogenic differentiation capacity, it was confirmed that the fetal MSC-derived MSCs having enhanced osteogenic differentiation capacity uniformly expressed CD73, CD44, CD29, CD105, and CD166, which are representative surface markers of MSCs, in an early culture passage (P9), a middle culture passage (P13), and in a late culture passage (P17) regardless of addition of bFGF (FIG. 9).

In addition, as a result of identifying expression of surface markers of pluripotent cells by FACS analysis in order to identify MSC characteristics of the fetal MSC-derived MSCs having enhanced osteogenic differentiation capacity, it was confirmed that the fetal MSC-derived MSCs having enhanced osteogenic differentiation capacity did not express SSEA-3 and TRA 1-81, which are representative surface markers of pluripotent cells, in an early culture passage (P9), in a middle culture passage (P13), and in a late culture passages (P17) regardless of addition of bFGF, but it was confirmed that cells expressing c-kit, as a fetal cell surface marker, were present, particularly, in the early cells under bFGF-free culturing conditions. Therefore, it was confirmed that expression of c-kit is one of the characteristics of the fetal MSC-derived MSCs having enhanced osteogenic differentiation capacity (FIG. 10).

In addition, as a result of identifying expression of surface markers of hematopoietic stem cells by FACS analysis in order to identify MSC characteristics of the fetal MSC-derived MSCs having enhanced osteogenic differentiation capacity, it was confirmed that the fetal MSC-derived MSCs having enhanced osteogenic differentiation capacity did not express CD14, CD24, CD34, CD45, and CD70, which are representative surface markers of hematopoietic stem cells, in an early culture passage (P9), in a middle culture passage (P13), and in a late culture passage (P17) regardless of addition of bFGF (FIG. 11).

In addition, as a result of identifying immunogenicity according to expression of HLA among the MSC characteristics of the fetal MSC-derived MSCs having enhanced osteogenic differentiation capacity, there was no expression of HLA class II (HLA-DR) at all as in the case of other MSCs, particularly, it was confirmed that HLA class I was hardly expressed in a bFGF-supplemented culture medium. Therefore, it was confirmed that a reduction in HLA class I expression in a bFGF-supplemented culture medium is one of the characteristics of the fetal MSC-derived MSCs having enhanced osteogenic differentiation capacity, indicating that the fetal MSC-derived MSCs having enhanced osteogenic differentiation capacity are very effective on increasing cell viability against immunological rejection in development of allogenic cell therapy products (FIG. 12).

### Example 7. Gene Expression Analysis

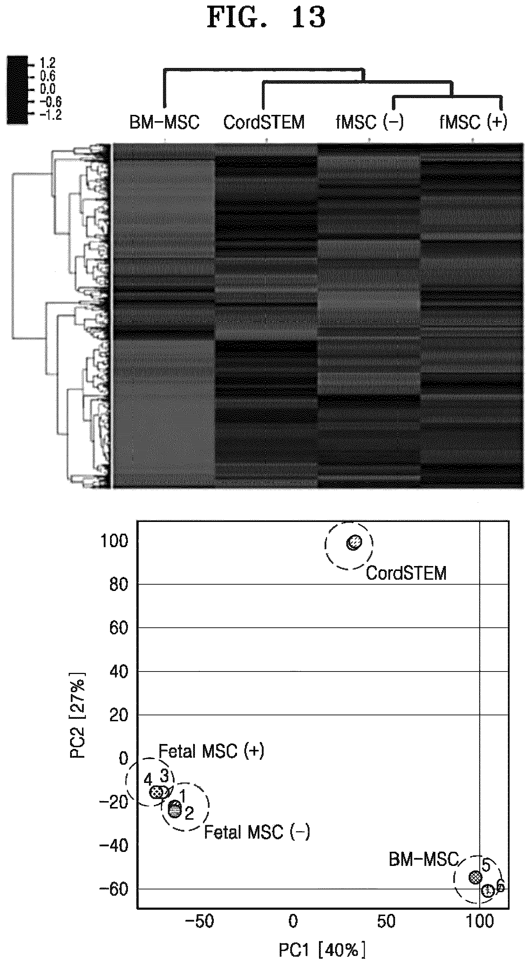

By performing RNA sequencing of MSCs derived from various sources, clustering heat maps and principal component analysis (PCA) results were obtained (FIG. 13).

The clustering heat maps and PCA results show relative expression (z-scores) of genes differently expressed in BM-MSCs, umbilical cord-derived MSCs (CordSTEMs), and fetal MSC-derived MSCs having enhanced osteogenic differentiation capacity in the presence or absence of bFGF in a culture medium.

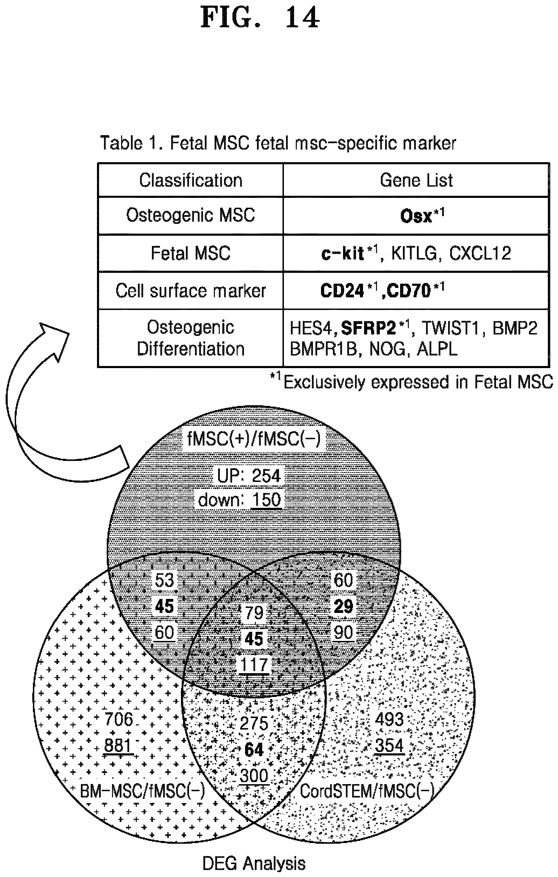

In addition, by analyzing differentially expressed genes, a Venn diagram in which genes differentially expressed in the MSCs derived from various sources overlap was obtained (FIG. 14). Table 1 of FIG. 14 shows a summary of marker genes specific to the fetal MSC-derived MSCs having enhanced osteogenic differentiation capacity exclusively (red) or highly (black) expressed in the fetal MSC-derived MSCs having enhanced osteogenic differentiation capacity.

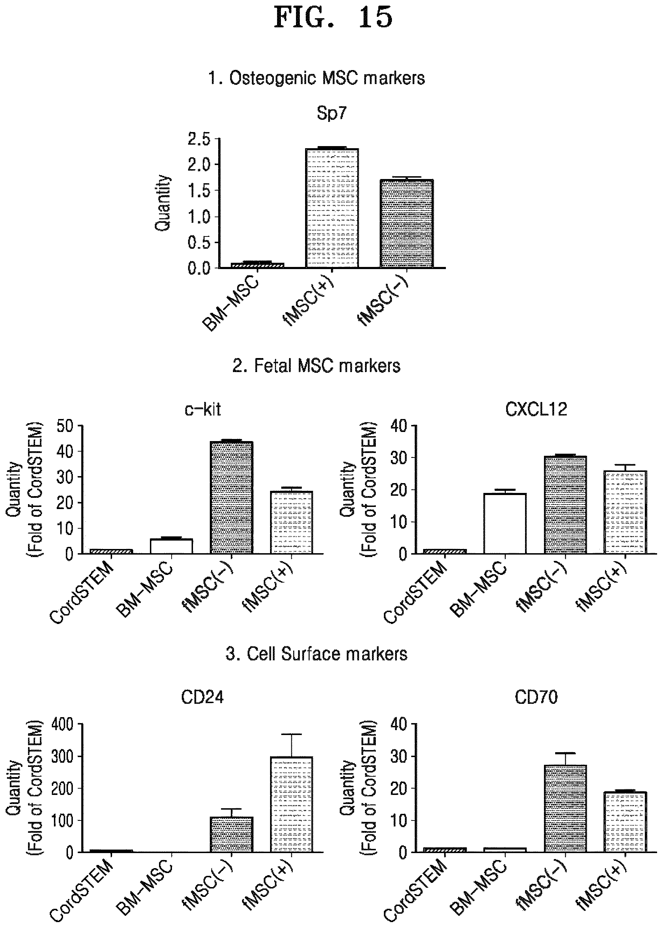

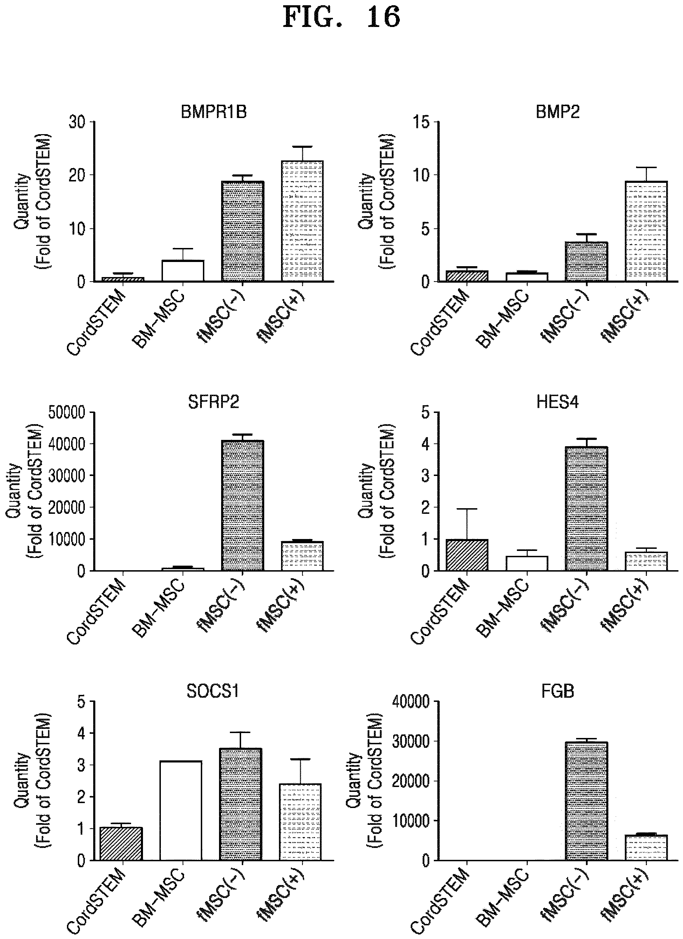

In addition, total RNA was prepared from MSCs derived from various sources, and by performing RT-PCR using primers specific to (1) osteogenic MSC marker genes, (2) fetal MSC marker genes, (3) cell surface marker genes, and (4) osteogenic differentiation marker genesshown in Table 1 of FIG. 14, expression of mRNA was identified (FIGS. 15 and 16).

As a result of analyzing gene expression in adult-derived MSCs (BM-MSCs), neonatal MSCs (Umbilical Cord MSCs) by RNA sequencing, expression of genes (markers) specific to the fetal MSC-derived MSCs having enhanced osteogenic differentiation capacity was confirmed. Fetal MSC-specific genes, which are not expressed in MSCs derived from other sources but expressed in the fetal MSC-derived MSCs having enhanced osteogenic differentiation capacity, are Osx (SP7), c-kit, CD24, CD70, and SFRP2. Therefore, it was confirmed that the fetal MSC is distinguished from other MSCs based on expression of these markers.

Among them, Osx (SP7) is known as an osteogenic MSC-specific marker in relation to osteogenic differentiation function, and SFRP2 is known to be involved in cell signaling that plays an important role in osteogenic differentiation.

In addition, C-Kit is a marker specific to cells derived from fetuses and it was confirmed that other fetus-derived genes such as KITLG and CXCL12 were overexpressed in the fetal MSC-derived MSCs having enhanced osteogenic differentiation capacity, as compared to MSCs derived from other sources.

In addition, CD24 and CD70 are cell surface markers, specificity of the fetal MSC-derived MSCs having enhanced osteogenic differentiation capacity may be distinguished by these markers.

It was confirmed that osteogenic differentiation-related transcription factors, ligands, cell signaling components, and enzymes were overexpressed in fetal MSC-derived MSCs having enhanced osteogenic differentiation capacity (e.g., HES4, TWIST1, BMP2, BMPR1B, NOG, and ALPL).

### Example 8. Secretome Analysis

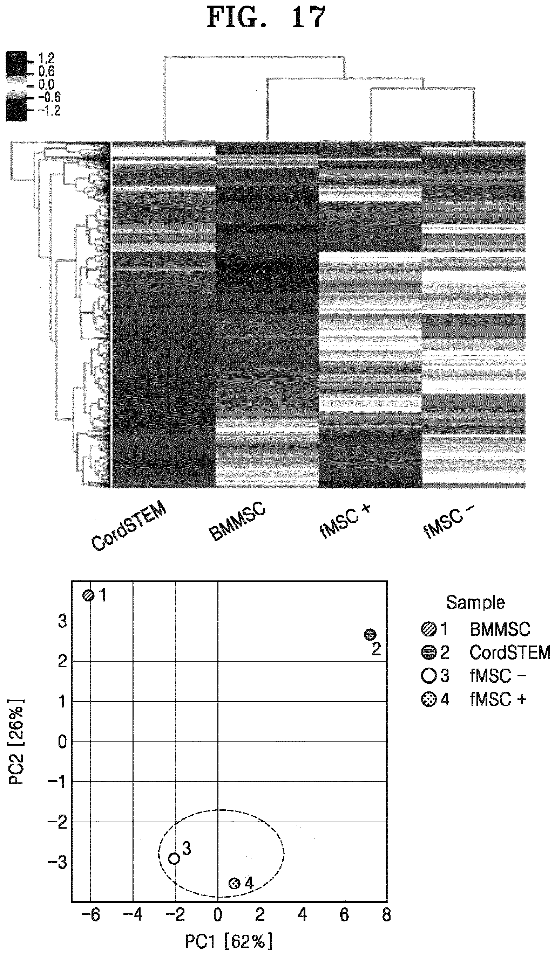

For secretome analysis, first, clustering heat maps were drawn and principal component analysis was performed (FIG. 17). Ray Biotech human cytokine array (Raybiotech#L-507, #L-493) was analyzed using serum-free media samples obtained from MSCs derived from various sources. The clustering heat map and PCA represent relative secretion of cytokines and growth factors differentially secreted from BM-MSCs, umbilical cord-derived MSCs (CordSTEMs), and fetal MSC-derived MSCs having enhanced osteogenic differentiation capacity in the presence or absence of bFGF in a culture medium.

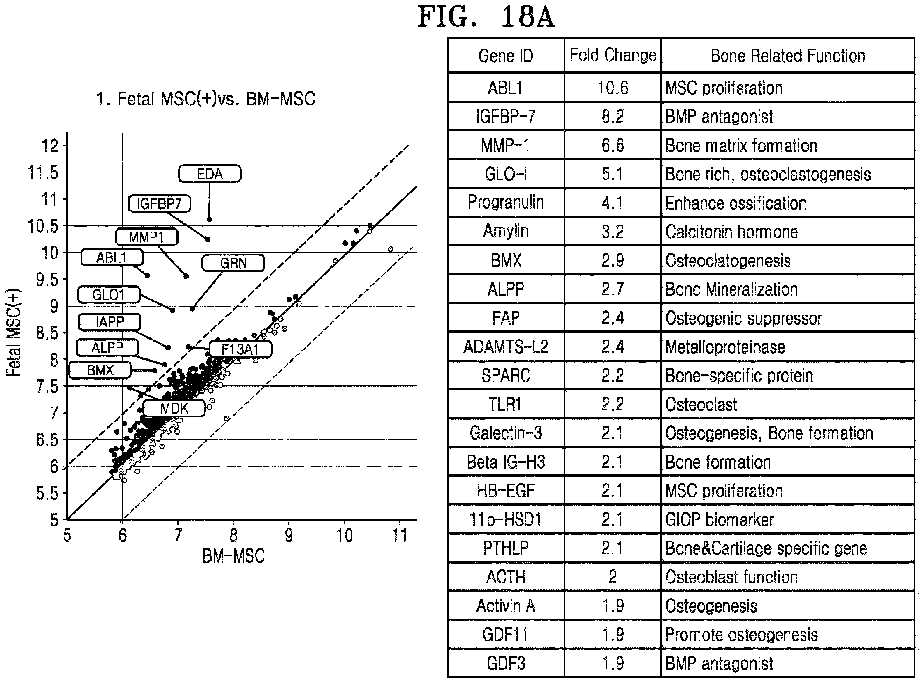

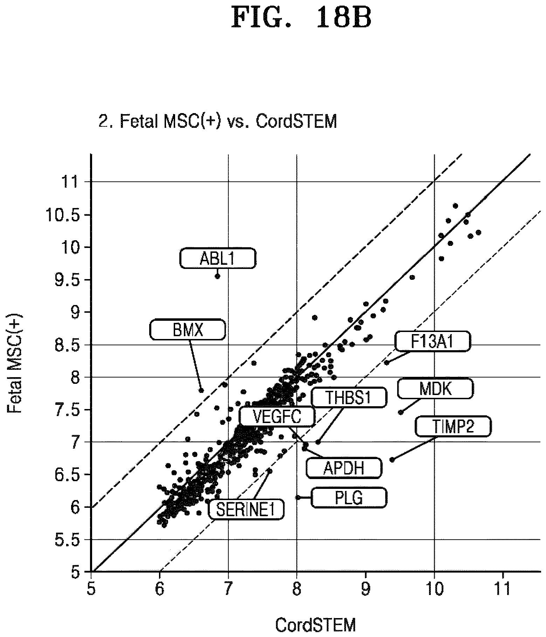

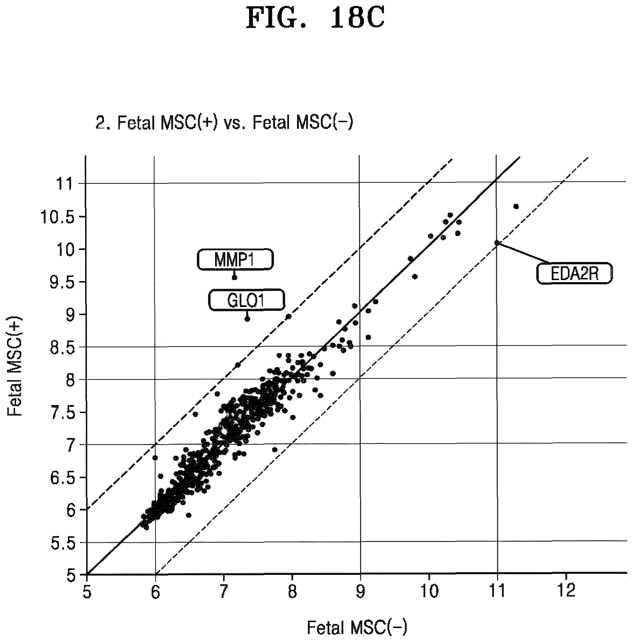

In addition, a scatter plot was identified (FIG. 18). The scatter plot shows the differentially secreted proteins and increased secretion of proteins, as compared to that of the fetal MSCs (+) was shown in red and decreased secretion was shown in green. A table of FIG. 18 shows a list of highly secreted genes and bone metabolism-related function thereof in the scatter plot.

As a result of analyzing secretion of proteins in adult-derived MSCs (BM-MSCs), neonatal MSCs (Umbilical Cord MSCs), and a culture medium by secretome analysis, secretion of proteins specific to the fetal MSC-derived MSCs having enhanced osteogenic differentiation capacity was confirmed. In comparison with MSCs derived from other sources, a plurality of proteins over-secreted from the MSCs having enhanced osteogenic differentiation capacity was confirmed (FIG. 18).

Particularly, in relation to proliferation of MSCs, osteogenic differentiation, or bone metabolism, a plurality of functional proteins such as cytokines and growth factors were expressed at high levels in the fetal MSC-derived MSCs having enhanced osteogenic differentiation capacity as summarized in the table of FIG. 18. Therefore, it was confirmed that the fetal MSC-derived MSCs having enhanced osteogenic differentiation capacity have excellent osteogenic differentiation function.

### Example 9. CD90 Heterogeneity and Osteogenic Potential

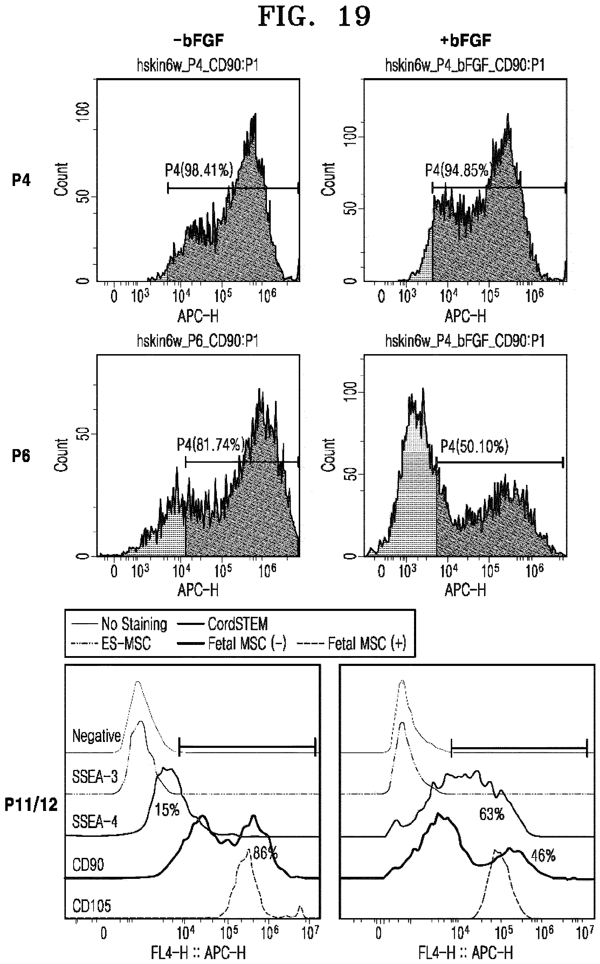

Because fetal MSC-derived MSCs having enhanced osteogenic differentiation capacity have CD90 heterogeneity, FACS analysis was performed to identify this property (FIG. 19). Specifically, MSCs (ES-MSCs and CordSTEMs) and fetal MSC-derived MSCs having enhanced osteogenic differentiation capacity were expanded into a culture medium in the presence or absence of bFGF. The cells were cultured in the shown passages (P4, P6, P11, and P12), and FACS analysis was performed by using antibodies specific to CD90, CD105, SSEA-3, and SSEA-4.

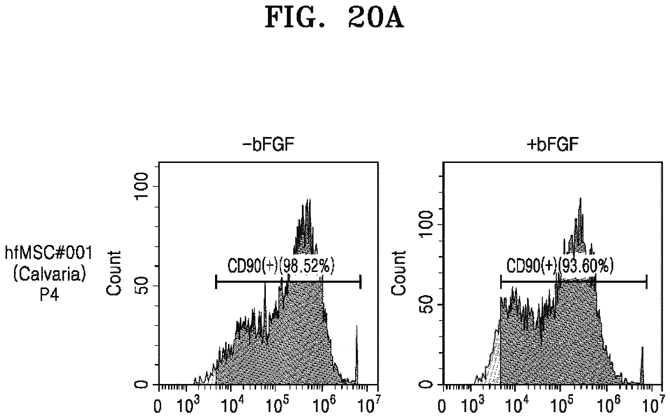

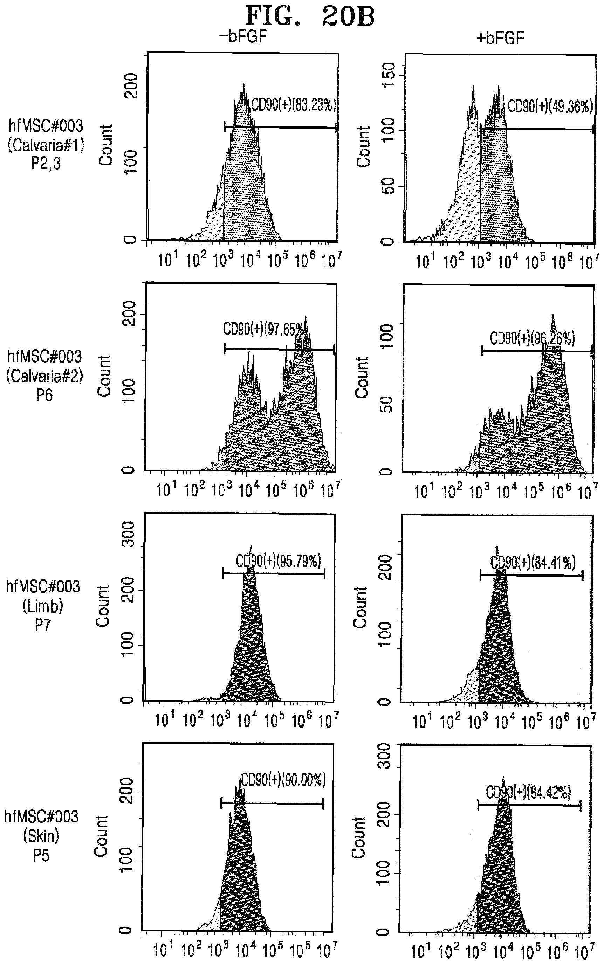

Specifically, MSCs derived from calvaria of skull tissue of a 8.5-week-old fetus (FIG. 20A) and MSCs derived from calvaria, limb, and skin tissue of a 7-week-old fetus (FIG. 20B) were analyzed by FACS. In the case of CD90 heterogeneity of MSCs having enhance osteogenic differentiation capacity, while CD90 heterogeneity was not identified in limb and skin-derived MSCs, CD90 heterogeneity was confirmed in bony tissue-derived MSCs.

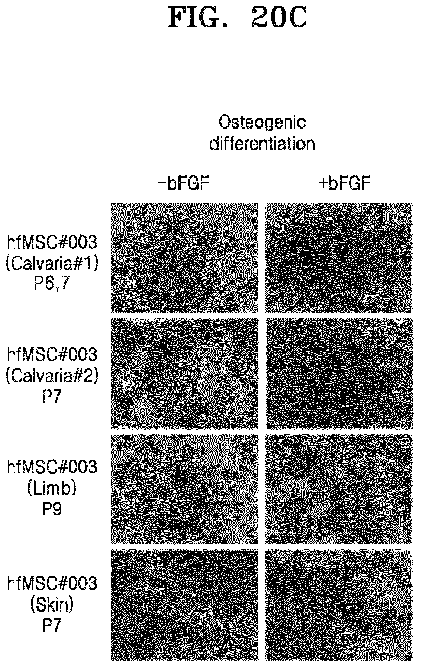

Meanwhile, it was confirmed osteogenic differentiation capacity of the calvaria-derived MSCs was the highest (FIG. 20C).

Based on the results described above, it was confirmed that the MSCs having enhanced osteogenic differentiation capacity were classified into cells with high CD90 expression levels and cells with low CD90 expression levels among the surface markers in the subculture process, and CD90 heterogeneity was significantly observed in MSCs derived from bony tissue (skull). It was confirmed that such a phenomenon was more significantly observed in the case of adding bFGF.

### Example 10. Protection and Regeneration of Bone by Fetal MSC-derived MSCs Having Enhanced Osteogenic Differentiation Capacity in Osteoporosis Animal Model

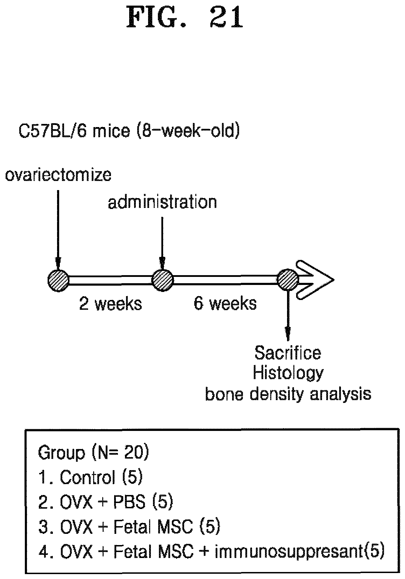

In order to identify effects of the fetal MSC-derived MSCs having enhanced osteogenic differentiation capacity on protecting and regenerating bones, an experiment was performed and FIG. 21 shows a plan of the experiment.

Specifically, 8-week-old female C57BL/6 mice were ovariectomized. After 2 weeks, 1 × 10⁶ of the fetal MSC-derived MSCs having enhanced osteogenic differentiation capacity or the same volume of PBS (100 µl) were injected into the mice via the tail vein. After 6 weeks from the transplantation of the fetal MSC-derived MSCs having enhanced osteogenic differentiation capacity, the mice were sacrificed. In the case of an immunosuppressed group, 10 mg/kg of cyclospholine A (CsA) was subcutaneously administered every day.

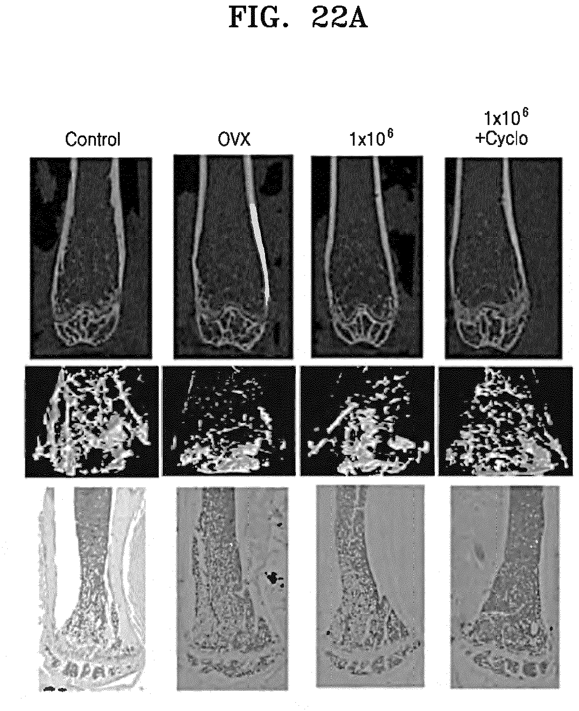

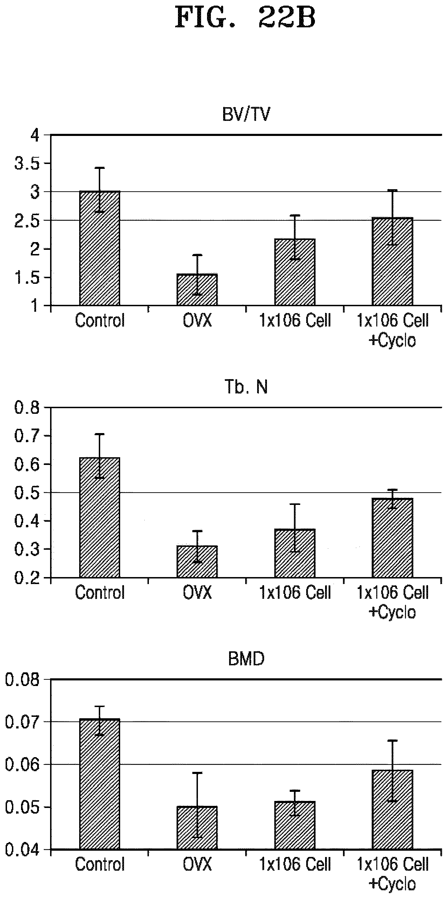

As a result, micro-computerized tomography images and bone densities are shown in FIG. 22. After the mice were sacrificed, femurs and tibiae were excised and immobilized with a 10 % PFA solution. Bone structural parameters were analyzed using a micro-CT system and H&E staining. In bone density and composition analysis, femurs and tibiae were scanned by using a micro-computed tomography system (SkyScan1173; Bruker-CT, Belgium) at 1-mm intervals. Bone volume (cm³), trabecular bone number, and bone density (mg/cm³) were calculated using CT analyzer software (CTAnMicro-CT software) (BV/TV: bone volume per tissue volume, Tb.N: trabecular bone number, and BMD: bone mineral density).

That is, an osteoporosis model was constructed by ovariectomizing to identify effectiveness of MSCs having enhanced osteogenic differentiation capacity on an animal model having a bone disease. As a result of administering the fetal MSC-derived MSCs having enhanced osteogenic differentiation capacity to the osteoporosis model via a vein, an increase in bone density, an increase in the number of osteocytes, and an increase in bone volume were confirmed. Therefore, it was confirmed that the fetal MSC-derived MSCs having enhanced osteogenic differentiation capacity may be used as a cell therapy product for indications of a bone disease such as osteoporosis, fracture, bone nonunion, osteogenesis imperfecta, and fibrous dysplasia.

In addition, it was confirmed that adipogenic differentiation of MSCs in bone marrow as one of mechanisms was reduced by administration of the fetal MSC-derived MSCs having enhanced osteogenic differentiation capacity in an ovariectomized model.

### Example 11. Protection of Estrogen Deficiency Syndrome

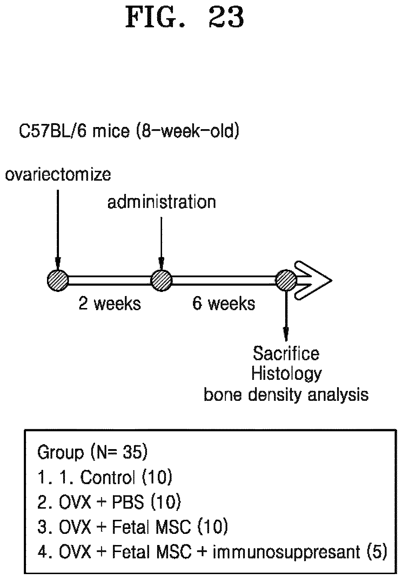

In order to identify effects of the fetal MSC-derived MSCs having enhanced osteogenic differentiation capacity on protecting an estrogen deficiency syndrome, an experiment was performed and FIG. 23 shows a plan of the experiment.

Specifically, 8-week-old female C57BL/6 mice were ovariectomized. After 2 weeks, 1 × 10⁶ of the fetal MSC-derived MSCs having enhanced osteogenic differentiation capacity or the same volume of PBS (100 µl) were injected into the mice via the tail vein. After 6 weeks from the transplantation of the fetal MSC-derived MSCs having enhanced osteogenic differentiation capacity, the mice were sacrificed. In the case of an immunosuppressed group, 10 mg/kg of cyclospholine A (CsA) was administered by using an osmotic pump (ALZET, USA).

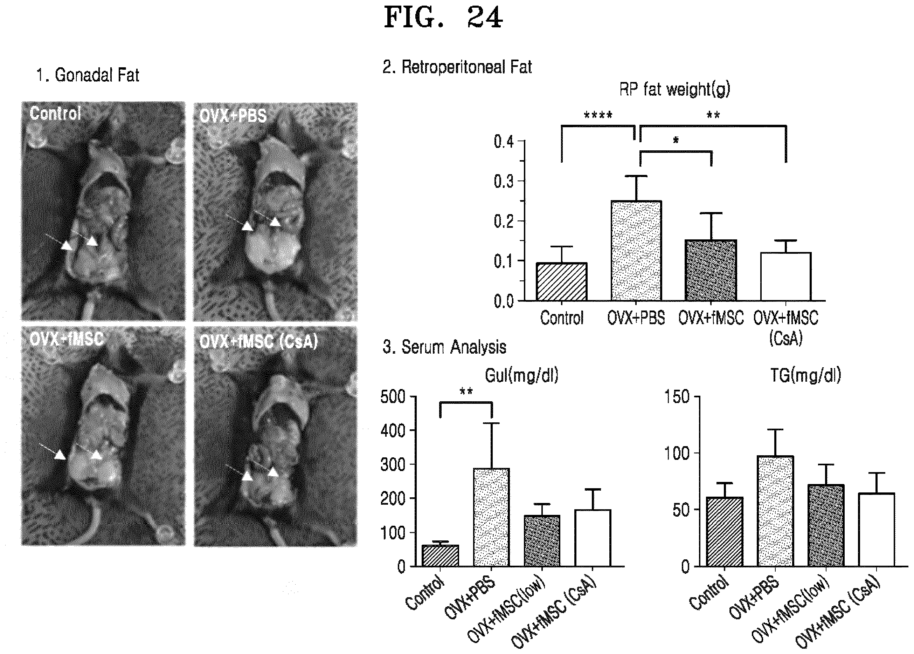

As a result, fatty tissue analysis results are shown in FIG. 24. After the mice were sacrificed, shapes and weights of fat pads (gonadal fat and retroperitoneal fat) were analyzed (One-way ANOVA test; P value ****; <0.0001, **; 0.0018, *; 0.0139). Blood sugar and total glyceride levels were analyzed using serum (One-way ANOVA test; P value **; 0.0025).

That is, an animal model was constructed by ovariectomizing to identify effectiveness of the fetal MSC-derived MSCs having enhanced osteogenic differentiation capacity in an estrogen deficiency syndrome animal model, and it was confirmed that obesity was caused by an increase in adipocytes that is a metabolic disorder, as one of estrogen deficiency syndromes caused by ovariectomizing.

It was confirmed that obesity, which was caused by an increase in adipocytes, was significantly reduced by intravenously administering the fetal MSC-derived MSCs having enhanced osteogenic differentiation capacity in the ovariectomized model. This, it was confirmed that the fetal MSC-derived MSCs having enhanced osteogenic differentiation capacity was available as a cell therapy product for indications of metabolic diseases (obesity and type II diabetes) which are the estrogen deficiency syndrome caused after menopause.

### Reference Examples

### Reference Example 1. Isolation and Culture of Human Fetal MSCs

After removing neural tubes from skull or limb tissue of ectopic or stillborn human fetuses, the skull tissue was added to a 50 ml-tube and HBSS was added. Then, blood was removed therefrom while mixing the mixture. The fetal tissue was cut into pieces using a razor blade and transferred to a 50 ml-tube together with 10 ml of HBSS. The skull tissue was softly dispersed using a 10 ml pipette and centrifuged at room temperature at 1200 rpm for 5 minutes. After removing a supernatant, 10 ml of a collagenase solution was added thereto, followed by incubation in a 37°C shaking incubator for 15 minutes. After tissue masses were sunken by centrifugation performed at room temperature at 1200 rpm for 3 minutes, 9 ml of a supernatant was transferred to a new 50 ml-tube using a pipette and the same amount of a DPBS solution supplemented with 10 % FBS was added thereto, followed by mixing using a pipette to block a collagenase enzyme. The resultant was filtered with a 100 µm strainer and centrifuged at 1500 rpm for 5 minutes at room temperature. Pellets were mixed with 1 ml of a culture medium (DMEM high glucose with 10 % FBS and 1 % Penicillin & Streptomycin). Cells were cultured at a density of 5 × 10⁵ per a T-25 flask.

### Reference Example 2. Differentiation Method of Human Fetal MSC

### (1) Osteogenic Differentiation

After culturing cells in a 24-well plate, a culture medium was replaced with one prepared by mixing a basal medium and a supplement of a StemPro osteogenesis differentiation kit (gibco, A10072-01) at 9:1, and adding penicillin/streptomycin thereto to be 1 %. The culture medium was replaced every 3 to 4 days. After immobilization with 4 % PFA, the cells were stained with a 2 % Alizarin Red solution for 30 minutes to identify calcium stained in red.

### (2) Adipogenic Differentiation

After culturing cells in a 12-well plate, a culture medium was replaced with one prepared by mixing a basal medium and a supplement of a StemPro osteogenesis differentiation kit (gibco, A10072-01) at 9:1, and adding penicillin/streptomycin thereto to be 1 %. The culture medium was replaced every 3 to 4 days. After immobilization with 4 % PFA, the cells were stained with an Oil Red solution for 15 minutes to identify oil drops.

### (3) Chondrogenic Differentiation

After cell pellets were cultured in a chondrogenic differentiation medium (DMEM-high glucose + 1 % FBS, 1X ITS+Premix, 10 ng/mL TGF-β1, 10 µM dexamethasone, 1 µM ascorbate-2-phosphate, 1 % sodium pyruvate, 1X NEAA, 1 % penicillin/streptomycin) for 21 days, and paraffin sections thereof were prepared and stained by H&E or PAS staining to identify chondrogenic differentiation.

### Reference Example 3. Experiment to Identify Differentiation Capacity of Human Fetal MSCs

### (1) Preparation of RNA and Synthesis of cDNA

After sufficiently dispersing harvested cells by adding 1 ml of trizol thereto, 200 µl of chloroform was added thereto and sufficiently mixed. After maintaining at room temperature for 2 to 3 minutes, the resultant was centrifuged with 12000 g for 15 minutes, and a colorless upper portion was transferred to a new tube. The same amount of 100 % ethanol was added thereto and mixed 5 to 6 times. The contents were transferred to a mini spin column of a GeneAll kit (GeneAll biotech, #304-150) and centrifuged with 10,000 g at room temperature for 30 seconds. After discarding the contents flowing out and 500 µl of a GW1 buffer was added thereto, centrifugation was performed under the same conditions. 700 µl of an RNW buffer was added thereto, followed by centrifugation. Then, the resultant was transferred to a new tube and RNA extraction was performed by using 50 µl of water. After RNA was extracted by centrifugation using 10,000 g for 1 minute and a concentration thereof was identified using a spectrometer (Nano drop), oligo dT/random primer, dNTP, RNA, and nuclease free water were added thereto by using a LaboPass cDNA synthesis kit (cosmogenetic, #CMRTK002) based on a total amount of 500 ng, followed by reaction at 42°C for 1 hour to synthesize cDNA.

### (2) Real time-PCR

After adding a SYBR green mix (Enzynomics, # RT500S), cDNA, primer(forward/reverse), RNase free water to each well of a 96 well reaction plate (applied biosystems, #N8010560), PCR cycle was proceeded by using an RT-PCR device (applied biosystems, viiA7). Then, expression levels of the genes were identified and compared by a comparative delta-delta-Ct or standard curve method.

### (3) FACS Analysis

Cells were isolated by using trypsin and collected by centrifugation. After washing the cells twice with PBS, the cells were dispersed in a FACS buffer at a concentration of 1 × 10⁵ cells/200 µl. Antibodies were maintained at 100x concentration and in a dark environment at 4°C for 30 minutes. The cells were washed with the FACS buffer and dispersed in 400 µl of the FACS buffer, and then analyzed using a FACS device.

### (4) Secretome Analysis

A 100 % confluence dish was prepared and washed twice with DPBS. 8 ml of a DMEM medium only supplemented with 1 % penicillin/streptomycin was added thereto, followed by incubation for 24 hours. All of a culture broth of a culture dish was transferred to a 15 ml-tube and centrifuged at 2000 rpm for 10 minutes, and then concentrations of target substances were identified and compared using a supernatant using an Antibody array (Raybiotech Human L1000 Antibody Array).

### (5) Ovariectomized Model (OVX model)

After 8-week-old female C57BL/6 mice were anesthetized through respiratory anesthesia, the back was incised, ovaries covered with fat were removed, and the incision site was sutured. After 2 weeks from the operation, various stem cells were administered via tail vein at a dose of 100 µl under various conditions while checking weights and states of the mice every week. After 6 weeks from the intravenous administration, the mice were sacrificed and tissue and blood samples were obtained and analyzed.

### (6) Karyotype Analysis

Fetal MSCs were cultured in culture media supplemented or unsupplemented with bFGF up to 8 passages (FIG. 8A) and 24 passages (FIG. 8B), respectively. Then, the cells were isolated by using trypsin. By Giemsa staining, G band of at least 10 cells in the metaphase stage were observed.

## Claims

1. A mesenchymal stem cell having enhanced osteogenic differentiation capacity, expressing at least one gene selected from the group consisting of Osx (SP7), c-kit, CD24, CD70, and SFRP2.

2. The mesenchymal stem cell of claim 1, wherein the stem cell heterogeneously expresses CD90.

3. The mesenchymal stem cell of claim 1, wherein the stem cell does not express HLA class I.

4. The mesenchymal stem cell of claim 1, wherein the stem cell overexpresses at least one gene selected from the group consisting of KITLG, CXCL12, HES4, TWIST1, BMP2, BMPR1B, NOG, and ALPL, as compared to umbilical cord Wharton's jelly-derived mesenchymal stem cells or adult bone marrow-derived mesenchymal stem cells.

5. The mesenchymal stem cell of claim 1, wherein the stem cell oversecretes at least one polypeptide selected from the group consisting of ABL1, IGFBP-7, MMP-1, GLO-1, Progranulin, Amylin, BMX, ALPP, FAP, ADAMTS-L2, SPARC, TLR1, Galectin-3, Beta IG-H3, HB-EGF, 11b-HSD1, PTHLP, ACTH, Activin A, GDF11, and GDF3, as compared to umbilical cord Wharton's jelly-derived mesenchymal stem cells or adult bone marrow-derived mesenchymal stem cells.

6. The mesenchymal stem cell of claim 1, wherein the stem cell is derived from tissue of an ectopic or stillborn fetus.

7. The mesenchymal stem cell of claim 6, wherein the tissue of the fetus is skull or calvaria tissue of a fetus.

8. A pharmaceutical composition for preventing or treating a bone disease, comprising the mesenchymal stem cell having enhanced osteogenic differentiation of claim 1.

9. The pharmaceutical composition of claim 8, wherein the bone disease comprises at least one selected from the group consisting of osteoporosis, fracture, bone nonunion, osteogenesis imperfecta, osteopenia, osteolytic metastasis, lumbar degenerative kyphosis, Paget disease, bone atrophy, osteomalacia, osteoarthritis, periodontal disease, rickets, aplastic bone disease, bone damage caused by bone metastasis of cancer cells, fibrous dysplasia, McCune Albright syndrome, bone deformity, and osteodysplasia.

10. The pharmaceutical composition of claim 8, wherein the bone disease comprises at least one selected from the group consisting of osteoporosis, fracture, fibrous dysplasia, McCune Albright syndrome, bone nonunion, and osteogenesis imperfecta.

11. A pharmaceutical composition for preventing or treating an estrogen deficiency syndrome, comprising the mesenchymal stem cell having enhanced osteogenic differentiation capacity of claim 1.

12. The pharmaceutical composition of claim 11, wherein the estrogen deficiency syndrome exhibits at least one symptom selected from the group consisting of facial blushing, perspiration, insomnia, nervousness, depression, dizziness, concentration disorder, short-term memory impairment, anxiety, memory loss, palpitation, muscle pain, joint pain, skin dryness and atrophy, vaginal dryness, vaginal atrophy, lower urethral atrophy, vaginitis, uterine atrophy, cystitis, pain in urination, urinary urgency, obesity, type II diabetes, hyperlipidemia, arteriosclerosis, fatty liver, sudden heart rate change, sleep disorder, loss of confidence and sexual desire, osteoporosis, atherosclerotic heart disease, venous thrombosis, irregular menstrual cycle, irregular menstrual flow, irregular menstrual period, hyperhidrosis, tinnitus, hypertension, digestive disorder, headache, cognitive dysfunction, fatigue, mood swing, Alzheimer's disease, dyspareunia, short-term memory disorder, decreased libido, blood flow disorder, and skin aging, caused by estrogen deficiency

13. The pharmaceutical composition of claim 11, wherein the estrogen deficiency syndrome exhibits at least one symptom selected from the group consisting of obesity and type II diabetes, caused by estrogen deficiency.

14. A health functional food for preventing or alleviating a bone disease, comprising the mesenchymal stem cell having enhanced osteogenic differentiation capacity of claim 1.

15. A health functional food for preventing or alleviating an estrogen deficiency syndrome, comprising the mesenchymal stem cell having enhanced osteogenic differentiation capacity of claim 1.
